# EUROPEAN PATENT SPECIFICATION

(11) **EP 3 316 896 B1**
(45) Date of publication and mention of the grant of the patent: **07.09.2022**
(21) Application number: 16734005.8
(22) Date of filing: 04.07.2016
(51) Int. Cl.: A61K 35/15, C12N 5/0784

(54) **DEVICE AND METHOD FOR OBTAINING IMMUNO-STIMULATORY ANTIGEN-PRESENTING CELLS**
VORRICHTUNG UND VERFAHREN ZUR HERSTELLUNG IMMUNSTIMULATORISCHER ANTIGEN-PRÄSENTIERENDER ZELLEN
DISPOSITIF ET PROCÉDÉ POUR L'OBTENTION DE CELLULES PRÉSENTATRICES D'ANTIGÈNE IMMUNOSTIMULATRICES

(30) Priority: 03.07.2015 US 201562188514 P; 28.07.2015 GB 201513278
(43) Date of publication of application: 09.05.2018
(73) Proprietor: Transimmune AG, 40212 Düsseldorf (DE); Yale University, New Haven, CT 06511 (US)
(72) Inventor: BAUER, Guenter, 24640 Schmalfeld (DE); EDELSON, Richard, Westport, CT 06880 (US); HENCO, Karsten, 40629 Düsseldorf (DE); DUCKWORTH, Justin, Weybridge Surrey KT13 8XB (GB)
(74) Representative: Bühler, Dirk
(86) International application number: PCT/EP2016/065725
(87) International publication number: WO 2017/005700

(56) References cited:
- WO-A1-2011/137365
- WO-A1-2014/106629
- TYLER S. DURAZZO ET AL: "Induction of monocyte-to-dendritic cell maturation by extracorporeal photochemotherapy: Initiation via direct platelet signaling", TRANSFUSION AND APHERESIS SCIENCE, vol. 50, no. 3, 1 June 2014 (2014-06-01), pages 370-378, XP055291862, GB ISSN: 1473-0502, DOI: 10.1016/j.transci.2013.11.008
- RICHARD L. EDELSON: "Mechanistic insights into extracorporeal photochemotherapy: Efficient induction of monocyte-to-dendritic cell maturation", TRANSFUSION AND APHERESIS SCIENCE, vol. 50, no. 3, 1 June 2014 (2014-06-01), pages 322-329, XP055291869, GB ISSN: 1473-0502, DOI: 10.1016/j.transci.2013.07.031

## Description

### FIELD OF THE INVENTION

The present invention relates to methods for producing immuno-stimulatory antigen-presenting cells. The present invention further relates to the use of such cells for treating patients suffering from hyper-proliferative disease such as cancer.

### BACKGROUND OF THE INVENTION

Antigen-presenting cells (APCs) including dendritic cells (DCs) can take up antigens, process and present them as peptide derivatives encased as components of major histocompatibility complexes (MHC) class I or II, via processes known respectively as cross-presentation or exogenous presentation. Dendritic cells migrate to regional lymph nodes where they are able to make contacts with members of the two major T cells subsets, CD8⁺ and CD4⁺ T cells that express TCRs specific for distinctive peptide-MHC complexes. The capacity of this APC-T cell interaction for enormous specificity for particular derived peptides is revealed by simple mathematics. For example, since 9-11 amino acid-long peptide fragments derived from tumor specific proteins are the length preferred for binding to class I MHC, and since there are 20 different types of amino acids, the potential enormous level of diversity of these complexes is 20¹¹. Class II-bound derived peptides are somewhat longer and hence provide even greater potential diversity and specificity. CD8 T cells, including cytotoxic T cells, recognize antigenic peptides presented in the context of Class I MHC, while CD4 T cells recognize peptides presented by class II MHC. If antigen-loaded dendritic cells are in a mature state in which they express costimulatory molecules, they will transmit an activation signal to T cells to proliferate and differentiate, which subsequently will launch an immune response against the antigen. The exquisite specificity of cellular immune system, composed of T cells with receptors for distinct MHC presented antigens and capable of a myriad of immunogenic and counterbalancing tolerogenic net immune responses can be wondrously empowering when working ideally, or dangerously destructive when dysfunctioning.

The underlying premise of this patent is that, rather than being so complex as to be only clinically manageable by brute force therapeutic drugs, it is possible to therapeutically collaborate with normal immuno-physiology to preferentially tune and tilt net T cell responses in the desirable direction required by the clinical situation. However, and this is key to the current invention, the natural balance between immunizing and tolerizing net effects in cellular immunotherapy, if tilted in the wrong direction, commonly introduces serious deleterious adverse reactions that are masked and hard to distinguish from simple disease progression. For example, inadvertently induced tolerance to cancer antigens may be misinterpreted as spontaneous cancer progression, while inadvertently induced immunity against tissue antigens may be misinterpreted as naturally uncontrolled auto-immunity or rejection of transplanted organs. This invention enables desirable polarization of these directional antigen-specific responses, so that immunity or tolerance can be individually maximized when desirable.

The anti-tumor immune response can be considered to form the interface between cancer and the immune system. Typically, an anti-tumor immune response begins with tumor cells in the tumor environment expressing a variety of tumor associated antigens (TAAs). TAAs may be mutated self-antigens, i.e. neoantigens resulting from somatic mutations (1, 2, 3), or wild-type self-antigens, which are over-expressed or selectively expressed by the tumor.

Cancer vaccination approaches aim at presenting TAAs on APCs such as DCs to launch an immune response against TAA-displaying tumor cells. For example, TAA-presenting mature DCs activate CD8⁺ cytotoxic and CD4⁺ helper T cells which are assumed to exit the lymph node and traffic back to the tumor microenvironment where they infiltrate and trigger tumor cell death by various death mechanisms.

However, if such DCs receive no maturation stimulus, they may instead induce tolerance ("down-regulation") via T cell deletion, anergy or production of T regulatory (Treg) cells. In fact, tumors are assumed to escape an anti-tumor immune-response by inducing immunosuppressive defense mechanisms that oppose T cell killing which otherwise would result in tumor shrinkage.

Against this background significant research activities have focused on leveraging the immune system's potential for an anti-tumor response including cancer vaccination with TAAs for prostate cancer (4), adoptive cell transfer therapy with chimeric antigen receptors (CARs) in which T cells are exogenously genetically modified to express an artificial tumor-specific (5), and the checkpoint inhibitors including CTLA-4-, PD-1- and PD-L1-antibodies.

Extracorporeal photopheresis (ECP) was developed for treating cutaneous T-cell lymphoma (CTCL) (6,7). During ECP, a patient's blood is apheresed. Red blood cells are re-infused immediately back into the patient, while the patient's plasma and leukocytes are passed through a polystyrene plate under low flow conditions that recapitulate forces in the post-capillary venule, damaged tissue or wounds. This first step which provides a physical force is commonly termed "plate passage." For some time during this plate passage procedure, mononuclear leukocytes, constituting about 5% of the patient's total body tumor cellular pool (since a majority of the malignant cells are tissue-localized) are exposed *ex vivo* to a photoactivated psoralen drug, 8-methoxy psoralen (8-MOP) in the presence of ultraviolet light type A (UVA), a combination therapy referred to as psoralen and UVA (PUVA). Since 8-MOP is inactive without UVA, and the excited 8-MOP state lasts only a tiny fraction of a second, the duration of activity of the drug and thus its apoptotic efficiency is controlled by exposure to UVA (8). In this manner, 8-MOP is induced to covalently crosslink base pairs of DNA. The original concept underlying ECP was the assumption that proliferating metastatic T-cells of CTCL would be destroyed and that symptoms of the disease could thus be alleviated. It, however, came as a surprise when CTCL was in fact cured in some patients.

Moreover since its FDA approval, ECP has interestingly also proven effective in the treatment not only of CTCL but also of solid organ transplant rejection (SOTR), graft-versus-host disease (GVHD) (9) and autoimmune diseases such as progressive systemic sclerosis (10, 11) and pemphigus vulgaris (12).

To explain the effectiveness of ECP in these seemingly contradictory therapeutic settings, it has been assumed that the physical force applied to monocytes during plate passage step and the interaction of these monocytes with platelets creates shear stress ultimately leading to synchronized, activated monocytes expressing various DC differentiation markers (14) which are indicative of immunostimulation. PUVA has the additional effect of preferentially inducing massive, slow apoptosis in the lymphocyte population (15, 16, 34). This could explain the efficacy of ECP when treating CTLC patients. PUVA-induced apoptosis of lymphocytes may provide antigen sources for both stimulatory DCs. DCs would then internalize these peptides, display them on class I or II major histocompatibility complex (MHC) molecules, and ultimately activate the adaptive immune system's CD8⁺ cytotoxic T cells or CD4⁺ helper T cells, respectively (19).

The down regulation or immunosuppressive effect of ECP when treating e.g. GvHD may be explained by the effect of PUVA on DCs during ECP. 8-MOP and UVA have been shown to upregulate expression of DC "suppression" genes thus favoring the generation of tolerogenic or immunosuppressive DCs (15) and to play a role in inhibiting graft rejection (16,17).

Giving these complexities, here is continuing interest in deciphering the events underlying ECP and harnessing them for anti-tumor therapy.

### OBJECTIVES AND SUMMARY OF THE INVENTION

One objective of the present invention is to provide methods for producing immuno-stimulatory autologous dendritic cells displaying disease-associated antigen.

Another objective of the present invention is to provide methods for producing immuno-stimulatory autologous antigen-presenting cells, preferably immuno-stimulatory autologous dendritic cells displaying disease-associated antigens.

Another objective of the present invention is to provide methods for producing immuno-stimulatory autologous antigen-presenting cells such as dendritic cells in an extracorporeal amount of blood obtained from a patient.

Yet another objective of the present invention is to prepare immune stimulatory autologous dendritic cells without intrinsically preparing in parallel tolerance inducing autologous dendritic cells.

Yet another objective of the present intervention is to separate the antigen forming step from the monocyte activation step.

Yet another objective of the present invention relates to the use of such immuno-stimulatory autologous antigen-presenting such as dendritic cells for treating patients suffering from diseases such as cancer.

These and other objectives as they will become apparent from the ensuing description hereinafter are solved by the subject matter of the independent claims.

Some of the preferred embodiments of the present invention form the subject matter of the dependent claims. Yet other embodiments of the present invention may be taken from the ensuing description.

In a first aspect, the description relates to a method for obtaining antigen-presenting cells displaying disease-associated antigens, said method comprising at least the steps of:
a) providing an extracorporeal sample of disease-effector cells and subjecting said disease-effector cells to apoptotic agents to release disease-associated antigens or providing isolated disease-associated antigens,
b) providing an extracorporeal quantity of a mammalian subject's blood sample comprising monocytes,
c) activating of said monocytes to induce their differentiation into immuno-stimulatory autologous APCs in the absence of apoptotic agents,
d) combining said immuno-stimulatory autologous APCs of step c) with said apoptotic disease-effector cells or isolated disease-associated antigens of step a).

As regards this first aspect, steps a) to c) need not be performed in this order. One may for example first perform the activation step of monocytes and then the step of subjecting disease-effector cells to apoptotic agents to release disease-associated antigens or providing isolated disease-associated antigens before combining them subsequently. However, they should be performed separate from each other in the sense that activation of monocytes does not take place simultaneously in the same sample where apoptosis is induced in disease-effector cells or where disease-associated antigens are added. The method is therefore considered to be a discontinuous process as induction of apoptosis and activation of monocytes is separated, i.e. do not take place simultaneously in the same sample. Activation of monocytes should in particular not take place simultaneously in the presence of 8-MOP/UVA. In a preferred embodiment, the monocytes of the "separate" preparation or "separately from step a)", or any further differentiated progeny cells thereof, such as immunostimulatory autologous APCs, are not contacted with an apoptotic agent, such as 8-MOP/UVA in any step of the inventive method. Thus, in this embodiment, the "separate" monocytes are prepared and activated in the absence of an apoptotic agent and after combining these monocytes with the apoptotic disease-effector cells or isolated disease-associated antigens, the activated monocytes (i.e. the immuno-stimulatory autologous APCs) are not contacted with an apoptotic agent.

One may use cytotoxic agents as used in chemotherapy including taxanes including docetaxel and paclitaxel, anthracyclines, cyclophosphamide, vinca alkaloids, cisplatin, carboplatin, 5-fluoro-uracil, gemcitabine, capecitabin, navelbine or zoledronate, or biologic tumor targeting agents such as anti-tumor monoclonal antibodies, including those delivering toxins such as Denileukin, Diftitox as apoptotic agents. In case of such apoptotic agents it may be necessary to remove the apoptotic agent before combining the apoptotic disease effector cell or disease-associated antigens with the immuno-stimulatory autologous APCs in step d). A preferred apoptotic agent is the combination of 8-MOP and UVA exposure (also referred to herein as PUVA) given that removal of 8-MOP may not be necessary as the apoptotic efficacy of 8-MOP can be switched by avoiding exposure to UVA in step c).

In one embodiment the steps of this first aspect take place outside the human or animal body.

In a second aspect, the description relates to a method for obtaining antigen-presenting cells displaying disease-associated antigens, said method comprising at least the steps of:
a) providing an extracorporeal sample of disease-effector cells and subjecting said disease-effector cells to apoptotic agents to release disease-associated antigens or providing isolated disease-associated antigens,
b) providing an extracorporeal quantity of a mammalian subject's blood sample comprising monocytes,
c) combining said apoptotic disease effector cells or isolated disease-associated antigens of step a) with said monocytes of step b),
d) activating of said monocytes in the mixture of step c) to induce their differentiation into immuno-stimulatory autologous APCs in the absence of apoptotic agents.

As for the first aspect the steps of subjecting disease-effector cells to apoptotic agents to release disease-associated antigens or providing isolated disease-associated antigens and activating monocytes should be performed separate from each other in the sense that activation of monocytes does not take place simultaneously in the same sample where apoptosis is induced in disease-effector cells or where disease-associated antigens are added. The method is therefore considered to be a discontinuous process as induction of apoptosis and activation of monocytes is separated, i.e. do not take place simultaneously in the same sample. Activation of monocytes should in particular not take place simultaneously in the presence of 8-MOP/UVA. In a preferred embodiment, the monocytes of the "separate" preparation or "separately from step a)" or any further differentiated progeny cells thereof, such as immunostimulatory autologous APCs, are not contacted with an apoptotic agent, such as 8-MOP/UVA in any step of the inventive method. Thus, in this embodiment, the "separate" monocytes are prepared in the absence of an apoptotic agent and after combining these monocytes with the apoptotic disease-effector cells or isolated disease-associated antigens, the cells are not contacted with an apoptotic agent.

As used herein, the term "apoptotic agent" refers to any agent or external condition or combination thereof in an effective amount to induce apoptosis in cells in vitro. The skilled person knows how to determine whether or not cells are apoptotic by standard assays such as FACS, Annexin V, caspase assay, mitochondrial transmembrane potential change assay etc.

One may use cytotoxic agents as used in chemotherapy including taxanes including docetaxel and paclitaxel, anthracyclines, cyclophosphamide, vinca alkaloids, cisplatin, carboplatin, 5-fluoro-uracil, gemcitabine, capecitabin, navelbine or zoledronate, or biologic tumor targeting agents such as anti-tumor monoclonal antibodies, including those delivering toxins such as Denileukin Diftitox as apoptotic agents. As regards this second aspect, PUVA is preferred as an apoptotic agent given that removal of 8-MOP may not be necessary as the apoptotic efficacy of 8-MOP can be switched by avoiding exposure to UVA in step c).

In one embodiment the steps of this second aspect take place outside the human or animal body.

In an embodiment that is preferred throughout the description for the first and second aspect said disease effector cells are tumor cells and said disease-associated antigens are tumor-associated antigens (TAAs). Such tumor cells or TAAs may be obtained preferably from solid tumors. Even though tumor cells or TAAs may be obtained from allogenic sources, it is preferred that the tumor cells or TAAs are of autologous origin.

In an embodiment that is also preferred throughout the description for the first and second aspect the autologous APCs are autologous dendritic cells.

In another embodiment that is preferred throughout the description for the first and second aspect the autologous APCs display TAAs such as autologous dendritic cells displaying TAAs.

In another embodiment that is preferred throughout the description for the first and second aspect, the mammalian blood sample, disease effector cells and disease-associated antigens are of human origin. Thus the description specifically contemplates for example the use of human blood samples, human tumor cells and human TAAs.

In another embodiment that is preferred throughout the description for the first and second aspect, the disease effector cells and disease-associated antigens, preferably the tumor-effector cells and tumor-associated antigens are obtained from solid tumors or cells shed from such solid tumors or tumor cells isolated from blood, spinal fluid or lymph.

In another embodiment that is preferred throughout the description for the first and second aspect, all disease effector cells, disease-associated antigens, blood samples, monocytes, etc. are outside the human or animal body when being used for the methods described herein.

In a third aspect, the description relates to a method for obtaining globally activated monocytes and/or antigen-presenting cells said method comprising at least the steps of:
a) providing an extracorporeal quantity of a mammalian subject's blood sample comprising monocytes,
b) activating of said monocytes to induce their differentiation into globally activated monocytes and/or immuno-stimulatory autologous APCs in the absence of apoptotic agents.

In one embodiment the steps of this third aspect take place outside the human or animal body.

In this aspect monocytes are activated outside the human or animal body to become initially globally activated monocytes and subsequently antigen-presenting cells without getting into contact with apoptotic disease-effector cells or disease-associated antigens outside the human or animal body. Thus, after the monocytes have been activated there is no combination or co-incubation step with apoptotic disease-effector cells or disease-associated antigens before re-introduction of the activated monocytes into the human brain and the body. Such activated monocytes which have been obtained in the absence of an apoptotic disease effector cell or disease-associated antigen can, for example, be reintroduced into a patient undergoing chemotherapy and/or gamma-irradiation therapy. Such cells may thus be particularly useful for treatment of tumors which are not resectable and where tumor-associated antigens are released in the human or animal body through chemotherapy and/or gamma-irradiation.

In another embodiment that is preferred throughout the description for the first and second aspect, monocytes are activated and induced to differentiate into immuno-stimulatory autologous APCs such as dendritic cells without the need for addition of a molecular cocktail comprising cytokines such as e.g. IL-4, GM-CSF, LPS, IFN-γ, IL-1β and/or TNF-α.

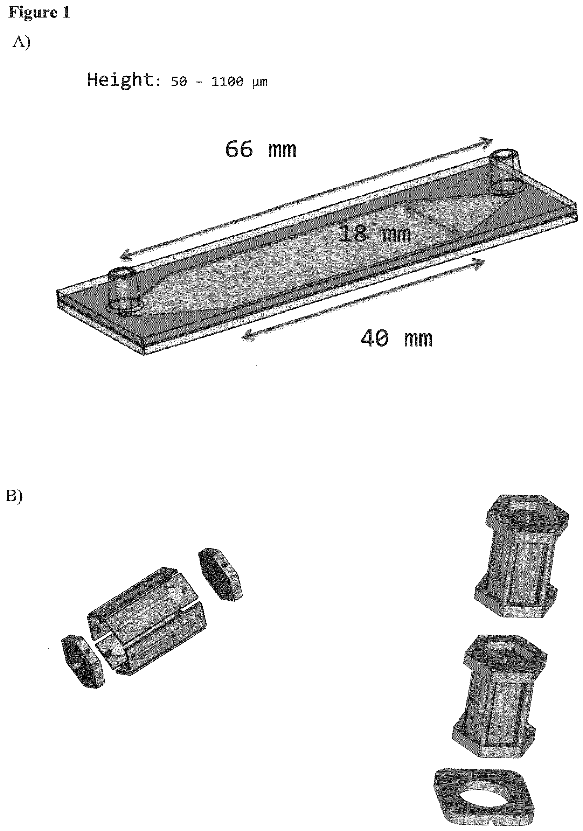

In another embodiment that is preferred throughout the description for the first, second and third aspect, the monocytes are activated and induced to differentiate into immuno-stimulatory autologous APCs by passing the cells in the absence of an apoptotic agent and in particular in the absence of PUVA through a device having flow chamber of dimensions comparable to those depicted in Figure 1. Such flow chambers will have a volume in the range of 0.2 ml to 5 ml. The height of such flow chambers may be in the range of about 20 µm to about 2000 µm, preferably in the range of about 50 µm to about 1000 µm.

In a fourth aspect described herein, immuno-stimulatory autologous APCs such as dendritic cells or globally activated monocytes as they are obtainable by the methods of the first, second aspect and third aspect and the embodiments thereof are for use in curative or preventive treatment of patients suffering from disease being mediated by the disease-effector cells or disease-associated antigens. Such immuno-stimulatory autologous APCs are thus in particularly considered for use in preventive or curative treatment of tumors, preferably solid tumors.

Further embodiments will be described hereinafter.

### FIGURE LEGENDS

- Figure 1: A) depicts a flow chamber as used in Experiments 1, 2 and 3. B) depicts one option of assembling flow chambers depicted in A9.
- Figure 2: depicts growth inhibition of YUMM tumors for individual mice. 8-MOP/UVA-treated Yumm 1.7 cells were mixed with PBMCs or PBS and passed through the same flow chamber and subjected to 8-MOP/UVA. Dashed lines depict tumor size of individual control group mice not being treated with flow chamber passaged PBMCs. Solid lines depict tumor size of individual treatment group mice being treated with flow chamber passaged PBMCs. Tumor volume was determined by cell counting.
- Figure 3: depicts combined growth inhibition of YUMM tumors of Figure 1 averaged across control and treatment groups. Dashed lines depict tumor size of control group where mice were not treated with flow chamber passaged PBMCs. Solid lines depict tumor size of treatment group where mice were treated with flow chamber passaged PBMCs. Tumor volume was determined by cell counting.
- Figure 4: depicts some of the treated mice of Experiment 1.
- Figure 5: depicts combined growth inhibition of YUMM tumors averaged across control and treatment groups. 8-MOP/UVA-treated Yumm 1.7 cells were mixed with PBMCs or PBS and passed through the same flow chamber but not subjected to 8-MOP/UVA. Dashed lines depict tumor size of individual control group mice not being treated with flow chamber passaged PBMCs. Solid lines depict tumor size of individual treatment group mice being treated with flow chamber passaged PBMCs. Tumor volume was determined by cell counting.

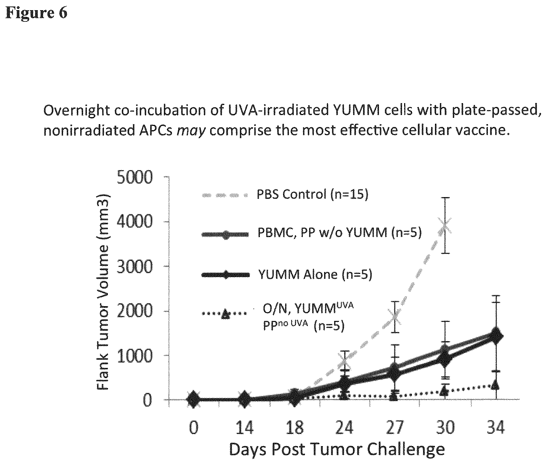

- Figure 6: depicts combined growth inhibition of YUMM tumors averaged across control and treatment groups The three treatment groups (five mice each) received only 8-MOP/UVA-treated flow chamber-passaged Yumm 1.7 (YUMM alone), only PBMCs which had been passed through the flow chamber but not subjected to 8-MOP/UVA (PBMC, PP w/o YUMM), PBMCs which had been passed through the flow chamber but not subjected to 8-MOP/UVA, and co-incubated with -MOP/UVA-treated flow chamber-passaged Yumm 1.7 cells overnight (Group 4, O/N YUMM^{UVA} PP^{noUVA}), or PBS. Tumor volume was determined by cell counting.
- Figure 7: A) depicts the geometry of a device used in some of the examples. B) depicts the geometry of an alternative device.
- Figure 8: depicts increase of expression of HLA-DR upon physical activation of monocytes through a device of Figure 7.
- Figure 9: depicts increase of FSC/SSC complexity upon physical activation of monocytes through a device of Figure 7.
- Figure 10: depicts increase of FSC/SSC complexity upon physical activation of monocytes by passing through a device of Figure 7.
- Figure 11: depicts increase of expression of HLA-DR, CD86, ICAM-1, PLAUR and or FSC/SSC complexity upon physical activation of monocytes through a device of Figure 7.
- Figure 12: depicts combined growth inhibition of YUMM tumors averaged across control and treatment groups. The treatment groups received PBMCs which had been passed through the flow chamber but not subjected to 8-MOP/UVA, and co-incubated with -MOP/UVA-treated flow chamber-passaged Yumm 1.7 cells overnight or not. Tumor volume was determined by measurement of the tumor volume.
- Figure 13: depicts growth inhibition of YUMM tumors averaged across control and treatment groups. 8-MOP/UVA-treated Yumm 1.7 cells were mixed with PBMCs or PBS and passed through the same flow chamber and were either subjected to 8-MOP/UVA or not. The study was repeated twice. Solid lines depict tumor size of individual treatment group mice. The tumor volume is given in arbitrary units.
- Figure 14: depicts growth inhibition of YUMM tumors averaged across control and treatment groups. 8-MOP/UVA-treated Yumm 1.7 cells were mixed with PBMCs or PBS and passed through the same flow chamber and were either combined with apoptotic agent-treated PBMCs or not. Solid lines depict tumor size of individual treatment group mice. The tumor volume is given in arbitrary units.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group, which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody, which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps unless indicated otherwise, i.e. the steps may be carried out simultaneously or there may be time intervals of seconds, minutes, hours, days, weeks, months or even years between such steps, unless otherwise indicated in the application as set forth herein above or below.

Technical terms are used by their common sense. If a specific meaning is conveyed to certain terms, definitions of terms will be given in the following in the context of which the terms are used.

The experimental data presented herein allow *inter alia* for the following observations. Using a novel device with a flow chamber of comparatively smaller dimensions than those used for ECP procedure it has been possible to reproduce the effects of ECP in a mice model of melanoma, i.e. for a solid tumor. To this end, apoptotic melanoma cells were co-incubated with peripheral blood mononuclear cells (PBMC) and subjected to PUVA treatment (i.e. application of 8-MOP and UVA) while passing the cells through said flow chamber. Subsequently, these cells were reinjected intravenously into the mice and an inhibition of tumor growth was observed. This, in essence, reproduced the series of steps and effects seen for patients suffering from CTLC and undergoing the classical ECP procedure. In another experiment, apoptotic melanoma cells were co-incubated with peripheral blood mononuclear cells (PBMC) but not subjected to PUVA treatment (i.e. application of 8-MOP and UVA) while passing the cells through said flow chamber. Nevertheless, tumor growth was inhibited when intravenously re-injecting these cells into mice.

It seems reasonable to conclude from these observations that the treatment of PBMC with PUVA which is a hallmark of the classical ECP procedure is in fact not necessary for producing autologous immuno-stimulatory antigen-presenting cells such as dendritic cells. Rather activation of monocytes and induction of differentiation of monocytes into mature autologous immuno-stimulatory antigen-presenting cells such as dendritic cells seems to be independent of PUVA treatment and can be achieved by passing monocytes through a device having a flow chamber as described herein optionally in the presence of platelets and plasma components. These cells seem nevertheless capable of taking up tumor-associated antigens (TAAs) which have been obtained separately by rendering tumor cells apoptotic, and mediating an anti-tumor response against solid tumors and thus enabling a curative treatment, i.e. treatment of existing tumors. It seems reasonable to conclude that these observations may also apply if such approaches were performed for other disease-mediating effector cells or other types of disease-associated antigens, for example for treatment of bacterial or viral infections. These experiments also suggest that it may be possible to produce such autologous immuno-stimulatory antigen-presenting cells such as dendritic cells and co-incubate them with TAAs or other disease-associated antigens for preventive treatment for example in situations where the disease has not yet developed.

By separating the generation of TAAs or other disease associated antigens from the generation of autologous immuno-stimulatory antigen-presenting cells such as dendritic cells, apoptosis of such developing autologous immuno-stimulatory antigen-presenting cells such as dendritic cells is reduced if not prevented thereby avoiding development tolerogenic dendritic cells which may negatively affect treatment outcome of cancers by allowing tumors to escape tumor surveillance.

Further it seems reasonable to assume in view of the presented data that separating the generation of apoptotic disease-effector cells such as tumor cells or disease-associated antigens such as TAAs by other means from the induction of autologous immuno-stimulatory antigen-presenting cells such as dendritic cells is not limited to using 8-MOP and UVA (which is also referred as PUVA herein) as apoptotic agent. Thus it may be possible to use for example known cytotoxic agents as used in chemotherapy such as taxanes including docetaxel and paclitaxel, anthracyclines, cisplatin, carboplatin, 5-fluoro-uracil, gemcitabine, capecitabin, navelbine or zoledronate, as well as direct radiation, such as γ-irradiation, strong UVB or UVC radiation, or very high temperatures for generating apoptotic disease-effector cells such as tumor cells or disease-associated antigens such as TAAs and to separate this step from the induction of autologous immuno-stimulatory antigen-presenting cells such as DCs, i.e. this induction step should take place in the absence of such apoptotic agents. This may require removal of e.g. cytotoxic agents before the induction step takes place. Against this background PUVA may of course be a particularly suitable apoptotic agent for the methods described herein as it allows performing the methods in a continuous manner given that the apoptotic potential of 8-MOP may be switched off by turning UVA off so that the induction step can take place in the presence of 8-MOP meaning that there is no need to remove 8-MOP before the induction step.

The data presented herein may further help explaining some effects observed for classical ECP. ECP treatment cycles are done weekly, biweekly, monthly or quarterly, whereas one treatment cycle comprises two ECP treatments on two consecutive days. The literature does not provide a rationale for this treatment schedule but given its broad worldwide acceptance and application it can be fairly assumed that this treatment schedule has been proven to provide a reasonable compromise between effective treatment and undesired side effects. In this context it is noteworthy that it has been observed for classical ECP that some patients suffering from CTLC and accompanying fungal infections showed improvement of CTLC while the fungal infection actually worsened as a consequence of ECP treatment. Further, it has been observed that in some CTLC patients ECP treatment may not be successful and even have a negative impact on the disease. One hypothesis that could explain these observations is that the 8-MOP/UVA induced apoptosis of cancerous T cells proceeds on a different timescale than the potential truncating effects of 8-MOP/UVA on DCs. Thus, during the first day of ECP treatment monocytes might become globally activated and even start differentiating into immuno-stimulatory DCs. At the same time apoptosis will be induced in cancerous T cells starting long-term antigen shedding. As ECP is a continuous process where the treated cells are continuously reintroduced into the patient's body and given that the next treatment schedule takes place on the next day, this type of "overnight incubation" may lead to formation of antigen-loaded immuno-stimulatory fully activated DCs. This cycle will be repeated on the second day and thereby potentially increase efficiency of treatment. However, at the same time the 8-and hope P/UVA treatment on the second day may either truncate differentiation of monocytes into immuno-stimulatory DCs and/or tolerogenize antigen-loaded DCs into tolerogenic DCs. This would of course in particular apply to antigen-loaded DCs which have already developed within the patient against other diseases such as accompanying fungal infections as these types of DCs would be subjected to a twofold treatment with 8-MOP/UVA. It could also explain why certain CTLC patients do not react favorably to ECP treatment, namely if such patients have already some antigen-loaded DCs which by the extended 8-MOP/UVA treatment would switch into tolerogenic DCs and thus have tolerogenizing effect against the tumor.

It is thus the continuous nature and the complex interplay of different factors of classical ECP that might explain its seemingly contradictory results.

With the data of the present disclosure at hand, where the global activation of monocytes and their differentiation into immuno-stimulatory DCs is separated from the interaction of apoptosis in cancer-derived cells (e.g. either by 8-MOP/UVA treatment or other cytotoxic agents), it seems reasonable to assume that the above-mentioned drawbacks of the prior art may be overcome. By generating immuno-stimulatory DCs and tumor-associated antigens or other disease-associated antigens in the discontinuous manner, both the truncation of developing DCs and the tolerogenizing of DCs as it is assumed to occur in ECP can be reduced.

With this new process it may become possible to achieve treatment of tumors including CTLC, but in particular solid tumors by obtaining PBMCs including monocytes only on one day during the treatment cycle rather than on two consecutive days. It may in fact allow reducing in addition the overall number of treatment cycles. The methods disclosed herein are characterized by the strict separation of obtaining activated monocytes and immuno-stimulatory DCs from the step of inducing apoptosis of cancer cells in particular by 8-MOP/UVA treatment. The methods disclosed herein which deviate from a hallmark feature of ECP, namely the simultaneous treatment of PBMCs and cancerous T cells during one phase of treatment cycle, may therefore allow improving CTLC treatment and even allow for treating solid tumors. Treatment of solid tumors may be achieved by either obtaining tumor cells directly from the tumor or by obtaining tumor-shedded cells from the bloodstream and subjecting it to apoptotic agents in a step separate from activation of monocytes and PBMCs.

Against this background and as mentioned above a first aspect described herein relates to a method for obtaining antigen-presenting cells displaying disease-associated antigens, said method comprising at least the steps of:
a) providing an extracorporeal sample of disease-effector cells and subjecting said disease-effector cells to apoptotic agents to release disease-associated antigens or providing isolated disease-associated antigens,
b) providing an extracorporeal quantity of a mammalian subject's blood sample comprising monocytes,
c) activating of said monocytes to induce their differentiation into immuno-stimulatory autologous APCs in the absence of apoptotic agents,
d) combining said immuno-stimulatory autologous APCs of step c) with said apoptotic disease-effector cells or isolated disease-associated antigens of step a).

As second aspect described herein relates to a method for obtaining antigen-presenting cells displaying disease-associated antigens, said method comprising at least the steps of:
a) providing an extracorporeal sample of disease-effector cells and subjecting said disease-effector cells to apoptotic agents to release disease-associated antigens or providing isolated disease-associated antigens,
b) providing an extracorporeal quantity of a mammalian subject's blood sample comprising monocytes,
c) combining said apoptotic disease effector cells or isolated disease-associated antigens of step a) with said monocytes of step b),
d) activating of said monocytes in the mixture of step c) to induce their differentiation into immuno-stimulatory autologous APCs in the absence of apoptotic agents.

These aspects will be discussed in more detail in the following for some of the following embodiments (flow chamber, activation by platelet and plasma components, etc.) with respect to rendering tumor cells apoptotic or using TAAs. Further, these aspects are discussed with respect to using PUVA for induction of apoptosis and activation of monocytes in the absence of PUVA. The autologous APCs are will be discussed in the context autologous dendritic cells with all of these components being preferably of human origin and even more preferably being entirely autologous. It needs however to understood that these embodiments are also considered for disease-effector cells or disease-associated antigens of non-human origin and/or non-tumor origin, etc.

As mentioned above the steps of the first and second aspect can take place outside the human or animal body.

The first and second aspect contemplates to use samples of patients being under medication including chemotherapy, radiation therapy or combinations thereof. While these medications provide for apoptosis of e.g. tumor cells in the patient with the consequence that the samples will comprise e.g. tumor-associated antigens, activation of monocytes in such samples is not considered to take place simultaneously with the apoptotic stimulus because the sample is not subjected to another apoptotic stimulus concurrently with activation of monocytes. Even in such samples derived from patients being under medication including chemotherapy, radiation therapy or combinations thereof, activation of monocytes is thus considered to take place in the absence of apoptotic agents. It can nevertheless be preferred that such patients are not under PUVA treatment.

The first and second aspect also consider to use samples obtained from patients not being under being under medication including chemotherapy, radiation therapy or combinations thereof

In a third aspect, the description relates to a method for obtaining globally activated monocytes and/or antigen-presenting cells said method comprising at least the steps of:
a) providing an extracorporeal quantity of a mammalian subject's blood sample comprising monocytes,
b) activating of said monocytes to induce their differentiation into globally activated monocytes and/or immuno-stimulatory autologous APCs in the absence of apoptotic agents.

In this aspect monocytes are activated outside the human or animal body to become initially globally activated monocytes and subsequently antigen-presenting cells without getting into contact with apoptotic disease-effector cells or disease-associated antigens outside the human or animal body. Thus, after the monocytes have been activated there is no combination or co-incubation step with apoptotic disease-effector cells or disease-associated antigens before re-introduction of the activated monocytes into the human brain and the body. Such activated monocytes which have been obtained in the absence of an apoptotic disease effector cell or disease-associated antigen can, for example, be reintroduced into a patient undergoing chemotherapy, radiation therapy such as gamma-irradiation therapy or combinations thereof. Such cells may thus be particularly useful for treatment of tumors which are not resectable and where tumor-associated antigens are released in the human or animal body through chemotherapy and/or radiation therapy. Such radiation therapy may include photon therapy such as X-ray therapy and gamma-irradiation therapy; and particle therapy such as electron-, proton-, neutron-, carbon ion-, alpha particle-, and beta particle-therapy.

This aspect thus does not differ from the first and second aspect in terms of activation of the monocytes, but in that activated monocytes are not contacted with apoptotic disease-effector cells or disease-associated antigens outside the human or animal body. The value of such activated monocytes is in the afore-mentioned scenario where the activated monocytes are directly introduced into a patient who is treated by chemotherapy and/or gamma-irradiation. Once entering the human or animal body, such activated monocytes can take up the tumor-associated antigens released by chemotherapy and/or gamma-irradiation and thereby become fully activated immuno-stimulatory antigen-presenting cells displaying tumor-associated antigens.

If monocytes are activated as described below by passing monocytes through a flow-chamber excerpting physical stress and/or inducing interaction with plasma and/or platelet combinations they will initially form what is considered by the present disclosure to be "globally activated monocytes". In other words, globally activated monocytes are cells obtainable by said method. In one embodiment, the term "globally activated monocytes" refers to cells that are derived from undifferentiated monocytes but are still less differentiated than immuno-stimulatory antigen-presenting cells.

Such cells can also be identified by increased expression of suitable molecular markers if compared to untreated monocytes. The data presented in Experiment 5 suggest that the process of obtaining immuno-stimulatory antigen-presenting cells seems to include a global monocyte activation step and a monocyte to immuno-stimulatory antigen-presenting cell (e.g. dendritic cell) differentiation step. These different steps seem to be traceable by molecular markers as and by Forward Scattering/Side Scattering Complexity (FSC/SSC Complexity), which is determinable by FACS analysis. The molecular markers may moreover be grouped according to their know function as e.g. molecular markers of antigen-presentation, molecular markers of cellular adhesion etc.. HLA-DR, CD86, and CD 80 may be considered to representative of antigen-presentation. PLAUR, and ICAM-1 may be considered to representative of cell adhesion.

A set of markers, which may be used for identification of globally activated monocytes and for differentiation vs immune-stimulatory antigen presenting cells or immune-suppressive antigen-presenting cells is found in below Table 1.

**Table 1**

| No. | Marker | NCBI Gene ID No. | mRNA REF | SEQ ID No. |
|---|---|---|---|---|
| 1 | ABCA1 | 19 | NM 005502.3 | 1 |
| 2 | ANXA5 | 308 | NM 001154.3 | 2 |
| 3 | CCL2 | 6347 | NM 002982.3 | 3 |
| 4 | CCL7 | 6354 | NM 006273.3 | 4 |
| 5 | CD68 | 968 | NM_001251.2, NM_001040059.1 | 5 |
| 6 | CRK | 1398 | NM_016823.3 NM_005206.4 | 6 |
| 7 | CXCL1 | 2919 | NM 001511.3 | 7 |
| 8 | CXCL2 | 2920 | NM 002089.3 | 8 |
| 9 | CXCL5 | 6374 | NM 002994.4 | 9 |
| 10 | CXCL16 | 58191 | NM 022059.3 | 10 |
| 11 | FAS | 355 | NM_152871.2, NM_000043.4, NM_152872.2 | 11 |
| 12 | IL10 | 3586 | NM 000572.2 | 12 |
| 13 | ITGA5 | 3678 | NM 002205.2 | 13 |
| 14 | ITGAV | 3685 | EF560727.1 | 14 |
| 15 | MMP9 | 4318 | NM 004994.2 | 15 |
| 16 | MSR1 | 4481 | NM_138715.2, NM_138716.2, NM_002445.3 | 16 |
| 17 | OLR1 | 4973 | NM_002543.3, NM_001172633.1, NM_001172632.1 | 17 |
| 18 | PLAU | 5328 | NM_002658.3, NM_001145031.1 | 18 |
| 19 | PLAUR | 5329 | NM_001005377.2, NM_001005376.2, NM_002659.3 | 19 |
| 20 | RAB7B | 338382 | NM_001164522.1, NM_177403.4 | 20 |
| 21 | RALA | 5898 | NM 005402.3 | 21 |
| 22 | SCARF 1 | 8578 | NM_003693.3, NM_145350.2 | 22 |
| 23 | SIRPA | 140885 | NM_001040022.1, NM_001040023.1 | 23 |
| 24 | THBS1 | 7057 | NM 003246.2 | 24 |
| 25 | TIMP1 | 7076 | NM 003254.2 | 25 |
| 26 | TNF | 7124 | NM 000594.3 | 26 |

Markers like HLA-DR, PLAUR and ICAM-1 as well as FSC/SSC complexity may moreover be considered to be indicative of global monocyte activation.

Further differentiation of globally activated monocytes into immuno-stimulatory antigen-presenting cells such as dendritic cells as it can be supported by contacting activated monocytes with apoptotic disease-effector cells or disease-associated antigens as referred to in the first and second aspect can be detected by increased expression of additionally e.g. CD83 (SEQ ID No.27), ADAM-Decysin (SEQ ID No.:28), CD40 (SEQ ID No.:29), CD80 (SEQ ID No.:30), LAMP-3 (SEQ ID No.:31), NEU1 (SEQ ID No.:32), LOX1 (SEQ ID No.:33), FPRL2 (SEQ ID No.:34), GPNMB (SEQ ID No.:35) and CCR7 (SEQ ID No.:36). Increased expression refers to a comparison with untreated monocytes. Such immuno-stimulatory antigen-presenting cells will in addition express the markers of globally activated monocytes.

As mentioned above, monocytes can be activated and induced to differentiate into immuno-stimulatory autologous APCs such as dendritic cells without the need for addition of a molecular cocktail comprising cytokines such as e.g. IL-4, GM-CSF, LPS, IFN-γ, IL-1β and/or TNF-α. This can have the advantage of obtaining immuno-stimulatory autologous APCs such as dendritic cells which are subjected to as less molecular interference as possible. An advantage of not using supra-pharmacologic concentrations of growth factors to produce therapeutic antigen presenting cells (APCs) is that cell differentiated under conditions that are not reproduced *in vivo* may not persist in the desired functional state once returned to the patient. In contrast, as is accomplished in ECP, APCs more physiologically differentiated extracorporeal from monocytes have a much better chance to function once returned to the patient.

It is further preferred throughout the description that the monocytes are activated by passing the cells in the absence of an apoptotic agent and in particular in the absence of PUVA through a device having flow chamber of dimensions comparable to those depicted in Figure 1. This applies also to the third aspect as mentioned above and discussed in more detail below.

Such flow chambers may have a width to height ratio of about 40:1 to about 400:1 such as about 50:1 to about 300:1 or about 50:1 to about 250:1. A flow chamber with such a width to height ratio will usually allow for efficient activation by ensuring that monocytes have a sufficient surface for attaching to and thereby getting activated. The flow chambers may for example have a height of about 20 µm to up to about 2000 µm of height, a width of about 5 mm to about 100 mm and length of about 40 mm to about 100 mm of length. Such flow chambers may have a volume in the range of 0.2 ml to 5 ml. For example such flow chambers may have a height of about 50 µm to up to about 1000 µm of height and a width of about 5 mm to about 50 mm with a width to height ratio of 50:1 to about 300:1.

The afore-mentioned width to height ratio may be a particularly preferred parameter when performing the methods described herein for activating monocytes. For such flow chambers one can use the flow rates and shear stress as mentioned hereinafter.

It is assumed that the activation of monocytes into first globally activated monocytes and then immuno-stimulatory antigen-presenting cells involves the direct or indirect adherence of monocytes to the walls of the flow chamber. Indirect adherence and activation may be in particularly mediated by plasma, plasma components and/or platelets which may be passed concomitantly with or separate from the monocytes through the flow chamber as will be described below. The relative dimensions of the flow chamber which may have dimensions of about 20 µm to up to about 2000 µm of height, of about 5 mm to about 30 mm of width and of about 40 mm to about 80 mm of length and the above-mentioned width to height ratio allows for efficient activation by ensuring that monocytes have a sufficient surface for attaching to and thereby getting activated.

The monocytes may e.g. be passed through said flow chamber with a flow rate to produce a shear force of about 0.01 to about 100.0 dynes/cm2, of about 0.05 to about 50.0 dynes/cm2, of about 0.1 to about 20.0 dynes/cm2 such as of about 0.2 to about 10.0 dynes/cm2.

Suitable shear forces allowing for activation of monocytes may be achieved by a flow chamber having the aforementioned width to height ratio of about 40:1 to about 400:1 such as about 50:1 to about 300:1 or about 50:1 to about 250:1.

As described in (34) activation of monocytes and induction of differentiation into immuno-stimulatory autologous antigen-presenting cells such as dendritic cells may be influenced by interaction of monocytes with activated platelets and/or specific plasma components in a situation where the monocytes experience physical force, which may be provided by a device as described hereinafter. Variation of process parameters thus include varying the nature, purity and concentrations of plasma components; the nature, purity and concentration of platelets; the order of steps by which plasma components and/or platelets are passed through and/or disposed on the flow chamber; the density by which the flow chamber is coated with plasma components and/or platelets, the flow forces of the extracorporeal quantity of the mammalian subject's blood sample and in particular the platelets and/or the monocytes are passed through the flow chamber of such a flow chamber, the temperature and/or time at which the extracorporeal quantity of the mammalian subject's blood sample and in particular the platelets and/or the monocytes are passed through the flow chamber of such a device, etc., the nature, purity and concentrations of additional factors such as cytokines are added to the extracorporeal quantity of the mammalian subject's blood sample and in particular to the monocytes, etc.

Activation and induction of monocytes may e.g. take place in the presence of platelets, plasma components or both platelets and plasma components, which have been passed before and/or concomitantly with said extracorporeal quantity of blood sample through said flow chamber.

Inducing differentiation of monocytes contained in an extracorporeal quantity of a mammalian subject's blood into immuno-stimulatory autologous antigen-presenting cells such as dendritic cells may work comparatively effective, if platelets which are comprised within said extracorporeal quantity of said mammalian subject's blood are activated and if the extracorporeal quantity of said mammalian subject's blood comprising at least monocytes in said device is treated by applying a physical force to the monocytes contained within said extracorporeal quantity of said mammalian subject's blood such that said monocytes are activated and induced to differentiate into immuno-stimulatory autologous antigen-presenting cells such as dendritic cells by binding to said activated platelets. However, activation of monocytes may also be achieved by direct interaction with plasma components, i.e. without interaction with activated platelets.

The steps of activating platelets and the subsequent activation and differentiation of monocytes into DC can be taken from (34) and include for example that (i) plasma components such as plasma proteins are passed through the flow chamber of the device so that these components adhere to the walls of the flow chamber, that (ii) platelets are passed through the flow chamber and are activated by binding to the plasma components and that (iii) monocytes-containing fractions such as an extracorporeal quantity of said mammalian subject's blood comprising at least monocytes are passed through the flow chamber and are activated for differentiation into antigen-presenting cells such as dendritic cells by binding to the activated platelets. It is, however, to be understood that these activities may also occur if the plasma fraction or plasma proteins or fragments thereof, the platelet fraction and the monocytes-containing fraction are passed simultaneously through the channels or channel-like structures as is the case for a whole blood fraction if obtained from the extracorporeal amount of blood as described below. It is further to be understood that the process may be performed even though not with same effectiveness by adhering only plasma components to the walls of the flow chamber and letting monocytes interact with the plasma components. Nevertheless, in the following these aspects will be discussed for an embodiment where steps (i), (ii), and (iii) are realized.

Plasma components may include proteins like fibrinogen or fibronectin, or fragments thereof like the gamma component of fibrinogen. Even though it seems that activation of platelets by plasma proteins such as fibrinogen and fibronectin is sufficient so that recombinantly expressed forms of these proteins are sufficient, it can be preferred to use plasma fractions which are obtained from the extracorporeal amount of blood sample and comprise these proteins as these plasma fractions have a more complex composition and may comprise all plasma components, which provide for an optimal activation of platelets.

Plasma protein fractions, plasma proteins or fragments thereof may be passed through the flow chamber, which may be made of plastic or non-plastic materials such as glass in order to adhere to the walls of the channels or channel-like structures. There is no requirement that the plasma fractions or plasma proteins are passed through the flow chamber at a specific physical force such as e.g. a specific pressure. However, in order to streamline the process, it is envisaged to pass the plasma fractions or plasma proteins through the flow chamber at a shear stress, which is comparable if not identical to the shear stress required for monocyte activation being described in more detail below. In general, the plasma fractions or plasma proteins are first pumped through the flow chamber to coat the surfaces thereof with plasma proteins, including fibronectin and fibrinogen. The flow rate of the plasma protein fractions, plasma proteins or fragments thereof through the flow chamber is controlled to obtain a desired level of protein adherence to the plastic surfaces. If desired, the flow can be stopped for a period of time and the plasma component can "soak" the surfaces of the flow chamber. By controlling the speed and timing of the pump that propels the plasma components through the flow chamber, the degree of coating of can be controlled. In one approach, the plasma fractions or plasma proteins are exposed to the surfaces of the flow chamber structures for a period between about 1 to 60 min, between about 1 to about 30 min, between about 1 to about 20 min, or between about 1 to about 10 min. To enhance plasma protein adherence to the surfaces of the flow chamber, the flow may be temporarily discontinued (for up to about 60 min), before resumption, or the flow rate may be slowed from the filling rate (up to 100 ml/minute) to as low as 5 ml/minute, during this phase of the procedure.

One can also envisage a scenario, where a device with a flow chamber is used for which the surfaces of the flow chamber have been pre-coated with e.g. purified plasma proteins or fragments thereof such as the gamma component of fibrinogen. Such pre-coated devices may be used if the whole process s conducted in a handheld device comprising a cartridge providing the flow chamber, which is configured for e.g. one time use. One can also envisage a scenario, where a device with a flow chamber is used for which the surfaces of the flow chamber have been pre-coated with e.g. platelets-rich plasma.

After the plasma fractions or plasma proteins or fragments thereof have been passed through the flow chamber and the surfaces thereof have been coated with plasma proteins, the platelet fraction may be passed by e.g. pumping into and through the flow chamber. The flow rate and residence time of the platelets within the flow chamber is selected to allow the platelets to bind to the plasma components or proteins or fragments thereof which have adhered before to the surfaces of the flow chamber and to thereby activated.

After the platelets have been passed through the flow chamber and have been activated by the plasma proteins or fragments thereof, which have been disposed on the surfaces of the flow chamber, the monocytes-containing fraction, e.g. the extracorporeal quantity of said mammalian subject's blood sample or the below-mentioned leukocyte or buffy coat fraction, which have been obtained from the extracorporeal amount of blood sample, is passed by e.g. pumping into and through the flow chamber, by applying a physical force leading ultimately to differentiation of monocytes into APCs. It is however to be understood that interaction of monocytes with activated platelets, platelet-derived factors or plasma components is not sufficient for activation and differentiation of monocytes without the application of a physical force at the same time.

It is also to be understood that the same events as described above will happen if an extracorporeal quantity of a mammalian subject's blood sample comprising platelets and plasma components is passed through the flow chamber. In this case, plasma components will adhere to the walls to the flow chamber and then activate platelets. However, in this scenario the process may be less controllable and account may be taken of this by increasing the residence time of the extracorporeal quantity of a mammalian subject's blood sample comprising platelets and plasma components in the flow chamber.

Application of a physical force for moving the monocytes-containing fraction through the flow chamber preferably may mean that a monocytes-containing fraction such as the extracorporeal quantity of a mammalian subject's blood sample is moved through the flow chamber under shear stress. Typically, monocytes-containing fraction may be passed through the flow chamber with a width to height ration as mentioned above under a shear force of about 0.01 to about 100.0 dynes/cm2, of about 0.05 to about 50.0 dynes/cm2, or of about 0.1 to about 20.0 dynes/cm².

In any case it should be made sure that a shear force is generated that allows binding of monocytes to activated platelets and differentiation of such activated monocytes into immuno-stimulatory DC. It is to be understood that activation of monocytes leads to immobilization, e.g. by interacting with activated platelets, platelets-derived factors or plasma components. In order to harvest the induced immuno-stimulatory autologous dendritic cells, one may increase the shear stress and/or may treat the immuno-stimulatory autologous dendritic cells with factors allowing disadherence from activated platelets, platelets-derived factors or plasma components by adding factors such as Plavic, Aspirin or other blood thinners.

Temperature is another factor to influence activation of monocytes and their differentiation into immuno-stimulatory autologous dendritic cells. The methods contemplated by the disclosure may be performed in a range of about 18°C to about 42°C, preferably in a range of about 22°C to about 41°C and more preferably in a range of about 37°C to about 41°C.

One parameter that can also be varied to tune activation of monocytes is the density by which the flow chamber is coated with plasma components and thus with platelets that bind to the plasma components. In general, the denser the surfaces of the flow chamber are coated with plasma components and platelets, the more efficient will be the monocyte activation.

Against this background, methods of the first, second and third aspect may be conducted, wherein activation of said monocytes and differentiation into immuno-stimulatory autologous antigen-presenting or dendritic cells can be influenced by the design and dimensions of the flow chamber, the flow rate at which the monocytes are passed through the flow chamber, the temperature at which the monocytes, platelets, platelets-derived factors and/or plasma components are passed through the flow chamber, the exposure of the monocytes to light, the order by which the monocytes, platelets, platelets-derived factors and/or plasma components are passed through the flow chamber, the density by which plasma components are coated to the surfaces of the flow chamber, the density by which platelets and/or platelets derived factors adhere to the surfaces and or to the plasma components of the flow chamber, and/or the density by which monocytes adhere to the platelets and/or platelets derived factors and or plasma components adhered to the surfaces of the flow chamber.

For such methods activation of said monocytes and differentiation into immuno-stimulatory autologous antigen-presenting or dendritic cells in the absence of apoptotic agents as described above comprises at least the steps of:
i. passing monocytes together with plasma components and platelets through said flow chamber, and
ii. applying a physical force to the monocytes such that said monocytes are activated and induced to differentiate into immuno-stimulatory autologous antigen-presenting or dendritic cells.

In other embodiments activation of said monocytes and differentiation into immuno-stimulatory autologous antigen-presenting or dendritic cells in the absence of apoptotic agents as described above of step c) of claim or step d) of claim 2 at least the steps of:
i. passing plasma components and platelets, which may be comprised within said extracorporeal quantity of said mammalian subject's blood sample or which may be provided separately from said mammalian subject's blood sample through said flow chamber such that they can adhere to the walls of said flow chamber,
ii. passing said monocytes contained within said extracorporeal quantity of said mammalian subject's blood sample through said flow chamber,
iii. applying a physical force to the monocytes such that said monocytes are activated and induced to differentiate into immuno-stimulatory autologous antigen-presenting or dendritic cells.

Activation of monocytes may be achieved by selecting conditions such as flow rate etc. that the monocytes can bind to the platelets and become thereby activated.

In yet other embodiments activation of said monocytes and differentiation into immuno-stimulatory autologous antigen-presenting or dendritic cells in the absence of apoptotic agents as described above comprises at least the steps of:
i. passing plasma components or plasma, which may be comprised within said extracorporeal quantity of said mammalian subject's blood sample or which may be provided separately from said mammalian subject's blood sample through said flow chamber such that they can adhere to the walls of said flow chamber,
ii. passing platelets or platelet components, which may be comprised within said extracorporeal quantity of said mammalian subject's blood sample or which may be provided separately from said mammalian subject's blood sample through said flow chamber such that they can interact with said plasma components of step i.,
iii. passing said monocytes contained within said extracorporeal quantity of said mammalian subject's blood sample through said flow chamber and
iv. applying a physical force to the monocytes such that said monocytes are activated and induced to differentiate into immuno-stimulatory autologous antigen-presenting or dendritic cells.

Activation of monocytes may be achieved by selecting conditions such as flow rate etc. that the monocytes can bind to the platelets and become thereby activated.

The flow chamber may have internal sub-structures to increase the surface area or to make the flow conditions less heterogeneous. The flow chamber may also be filled with particles to increase the surface area or to make the flow conditions less heterogeneous.

The material of the flow chamber may be plastic or non-plastic. If non-plastic materials are considered, one may use glass. If plastic materials are considered, one may use acrylics, polycarbonate, polyetherimide, polysulfone, polyphenylsulfone, styrenes, polyurethane, polyethylene, teflon or any other appropriate medical grade plastic. In a preferred embodiment of the present invention, the flow chamber is made from an acrylic plastic.

As mentioned the flow rate through flow chamber and thus the resulting shear stress will affect the differentiation of the monocytes into immuno-stimulatory autologous APCs such as dendritic cells. Aside from the flow rate, the design and the dimensions of the flow chamber may be varied to manipulate and even improve the application of a physical force to the monocytes. Further in order to increase the throughput of monocytes, devices as shown in Figure 1A and having the above-mentioned dimensions may be combined as shown in Figure IB.

The person skilled in the art is familiar how to obtain whole blood, a leukocyte fraction thereof or a buffy coat fraction thereof (see e.g. Bruil et al., *Transfusion Medicine Reviews* (1995), IX (2), 145-166) an include filtration, differential centrifugation. A preferred method relies on filters as they are available from e.g. Pall. Such filters may be incorporated into the device such that processing of the extracorporeal sample can be done in the handheld device. As a source one can also use e.g. blood of the umbilical cord. If one uses centrifugation, one may obtain whole blood through a syringe with e.g. a 17 or 18 gauge-gauge needle. Such a whole blood sample may be centrifuged to remove debris and other components. The whole blood sample may then be filtered through common filters, as they are available from Pall.

For obtaining a mononuclear leukocyte fraction, one may obtain a whole blood sample as described and then layer such a sample on e.g. Ficoll-Hypaque. Subsequently a centrifugation step is performed at e.g. about 100g to about 200g such as 180 g and the mononuclear leukocyte fraction can then be collected from the interface and washed with common buffers such as HBSS. The washed mononuclear leukocyte fraction can then be resuspended in serum-free cell culture medium such as RPMI-1640 medium (GIBCO). Other methods for obtaining mononuclear leukocyte fractions include elutriation, filtration, density centrifugation, etc..

One can also use apheresis and leukapheresis to obtain the desired blood samples. Apheresis such as leukapheresis may be performed as is known in the art. Thus, an extracorporeal quantity of blood such as 2.5 L to 61 may be obtained from a subject and treated by conventional leukapheresis to obtain three fractions, namely the plasma, the platelets and the buffy coats. The plasma, which contains proteins such as fibronectin and fibrinogen, is the lightest blood fraction, and therefore is the first portion of the blood selectively removed from the centrifuge and passaged through channels or channel-like structures. After the plasma has been pumped through the channels or channel-like structures and the surfaces thereof have been coated with plasma proteins, the second lightest component in the leukapheresis centrifuge, the platelet fraction, is pumped into and through the channels or channel-like structures. The third lightest fraction to be eluted from the leukapheresis centrifuge is the buffy coat, which contains the white blood cells, including the blood monocytes. The buffy coat including the monocytes is then pumped through the channels or channel-like structures. Blood samples may be obtained using the Therakos device, the Spectra cell separator (see Andreu et al., (1994), Transf Sci., 15(4), 443-454), or the Theraflex device from Macopharma.

As pointed out above, steps for the induction of APC formation seem to involve the activation of platelets by plasma components and the activation of monocytes by such activated platelets. In principle, one could pass a whole blood sample through the device under shear stress. The plasma components of such a sample will then bind to the surfaces of the flow chamber and allow for adherence and activation of platelets within such a sample by plasma-components. The monocytes of such a sample will then bind to the activated platelets and be activated themselves.

Similarly one may obtain combinations of the various components such as a platelet-rich plasma containing fraction which may be obtained by centrifuging a whole blood sample which has been obtained as described above at about 100 g to about 180 g such as about 150 g for about 10 min to about 20 min such as about 15 min to separate the debris of the whole blood sample. The platelet-rich plasma layer is then collected and recentrifuged at about 700 g to about 1000 g such as about 900 g for about 3 min to about 10 min such as about 5 min. The resultant pellet is then resuspended in serum-free cell culture medium.

However, in order to have control over the process, it may be desirable to first pass plasma components through the flow chamber and let them adhere, then platelets and then the monocytes-containing fraction. For this approach, it may be desirable to obtain a leukocyte fraction comprising a monocytes- or buffy-coat fraction comprising monocytes, which does not comprise plasma components and which does not comprise platelets. Such plasma- and platelet-free monocytes-containing fractions may be obtained as is described in the art. If leukocyte or buffy-coat fractions are obtained as described above, they will be sufficiently free of plasma or platelets for the purposes of the invention. For this approach, it may also be desirable to have platelet- and/or plasma-fractions.

Thus, the description contemplate to use platelets which have been separated from the extracorporeal quantity of said mammalian subject's blood before said extracorporeal quantity of said mammalian subject's blood is applied to said device. These platelets may then be passed through the flow chamber, which has been coated with plasma components such as fibronectin.

In another embodiment, the description considers to use plasma components, which have been separated from the extracorporeal quantity of said mammalian subject's blood before said extracorporeal quantity of said mammalian subject's blood is applied to said device. These plasma components may then be passed through flow chamber so that they can adhere.

Instead of using plasma components which have been obtained from the extracorporeal amount of blood, one may also use plasma components, which have been isolated from other sources such as e.g. by recombinant protein expression. Such plasma components include fibrinogen, fibronectin, P-selectin, and fragments thereof such as the gamma component of fibrinogen.

Instead of using plasma components which have been obtained from the extracorporeal amount of blood, one may use also either plasma components which have been isolated from other sources such as e.g. by recombinant protein expression. Such plasma components include fibrinogen, fibronectin, or P-selectin. One can also use fragments of plasma proteins such as the gamma component of fibrinogen which corresponds to amino acids 400-411 (SEQ ID NO.: 37), His-His-Leu-Gly-Gly-Ala-Lys-Gln-Ala-Gly-Asp-Val). This gamma component is shown by the data presented herein to be able to activate platelets. It can therefore be preferred to use plasma fractions, which at least, if not predominantly comprise fibronectin. Similarly, it can be preferred to use e.g. recombinantly expressed and/or purified fibronectin or the gamma component thereof to activate platelets.

The extracorporeal quantity of a mammalian subject's blood may typically be obtained from between 0.1% - 10% of total blood volume of the respective subject. The quantity of extracorporeal blood withdrawn and applied to the device may be whole blood. Alternatively, said extracorporeal quantity of said mammalian subject's blood may be obtained by isolating leukocytes from between 0.5^{∗} 10⁶-50^{∗}10⁶ mononuclear cells.

Activated monocytes and immuno-stimulatory antigen-presenting cells may be removed from the flow chamber by washing with e.g. buffer at increased flow rates. As activated monocytes and immuno-stimulatory antigen-presenting cells may have a tendency to directly or indirectly stick to the surfaces of the flow chamber, removal may be supported by gently rocking the flow chamber or by gently knocking at the flow chamber to support.

Once activated monocytes and immuno-stimulatory antigen-presenting cells have been obtained by for example method of the first aspect, they may be contacted with disease-effector cells or isolated disease-associated antigens. This contacting step may take the form of an incubation step for at least 12 hours and or at least 24 hours. This incubation step with the apoptotic disease-effector cells or the isolated disease-associated antigens may allow the immuno-stimulatory antigen-presenting cells to complete their maturation and to display the disease-associated antigens. Such an incubation step which may take the form of an overnight incubation can be particularly preferred for step d) of methods of the first aspect. Incubation can be performed under standard conditions, e.g. at 37° C and 5% CO₂ in standard mediums for culturing of human cells such as in RPMI-1640 medium (obtainable e.g. from GIBCO), supplemented with 15% AB serum (obtainable from e.g. Gemini BioProducts).

After incubation, such cells can be introduced into patients for use in treating the respective disease such as the respective tumor for which tumor-associated antigens had been incubated with activated monocytes and immuno-stimulatory antigen-presenting cells.

In this way mature TAA-presenting immune-stimulatory autologous antigen-presenting cells such as dendritic cells can be obtained without the need for rather expensive cocktails of cytokines for induction of monocyte to DC differentiation. Even though not necessary, it can be considered to cultivate such TAA-presenting antigen-presenting cells in a buffered culture medium with one or more cytokines, such as GM-CSF and IL-4, during the incubation period. Maturation cocktails (typically consisting of combinations of ligands such as CD40L, cytokines such as interferon gamma, TNF alpha, interleukin 1 or prostaglandin E2 or the factors mentioned above) may be added as well.

As already mentioned, autologous antigen-presenting cells which have been obtained by applying a physical force as described herein may be contacted with at least one TAA or a tumor sample to generate autologous TAA-displaying antigen-presenting cells such as dendritic cells. Even though the disclosure focuses on the generation of autologous TAA-displaying antigen-presenting cells, it needs to be understood that autologous antigen-presenting cells displaying other disease related antigens of e.g. bacterial, fungal or viral origin may be obtained along the same lines and that disease-associated antigen displaying antigen-presenting cells by also be achieved by the methods described herein.

Autologous antigen-presenting cells such as dendritic cells can thus e.g. be processed ex vivo, such as by loading them with immunogenic antigens, e.g. those expressed on apoptotic tumor cells. As mentioned this will lead to immuno-stimulatory TAA-displaying antigen-presenting cells. If an antigen is used, which is already expressed by cancerous tissue obtained from the subject, from whom the extracorporeal amount of blood was taken to generate immuno-stimulatory autologous antigen-presenting cells, the TAA-loaded, reintroduced immuno-stimulatory autologous antigen-presenting cells may launch an immune response against the cancer.

In one embodiment of the invention, the dendritic cells are loaded with immunogenic antigens, wherein the immunogenic antigen comprises a synthetic or recombinant peptide. In another embodiment, the immunogenic antigen comprises a nucleic acid or a chemical derivative thereof, preferably RNA, and more preferably mRNA or siRNA and even more preferably mRNA. In one embodiment, the RNA contains non-natural nucleobases (i.e. nucleobases other than A, U, C and G). The nucleic acid as described above may also be chemically modified, i.e. a chemical derivative thereof.

As mentioned above, a fourth aspect described herein thus relates to immuno-stimulatory autologous antigen-presenting cells such as dendritic cells as they are obtainable by the methods of the first and second aspect and their embodiments or to globally activated monocytes and/or immuno-stimulatory autologous antigen-presenting cells such as dendritic cells as they are obtainable by the methods of the third aspect and its embodiments for use in curative or preventive treatment of patients suffering from disease being mediated by the disease-effector cells or disease-associated antigens. Such immuno-stimulatory autologous antigen-presenting cells are thus in particularly considered for use in preventive or curative treatment of tumors, preferably solid tumors. The activated monocytes which are obtainable according to the first and second aspect and their embodiments or according to the third aspect and its embodiments differ in that activated monocytes according to the first and second aspect and their embodiments have been in contact outside the human or animal body with apoptotic disease-effector cells such as apoptotic tumor-effector cells and/or disease-associated antigens such as tumor-associated antigens before they are reintroduced into the human or animal body, while activated monocytes according to the third aspect and it's embodiments have not been in contact outside the human or animal body with such apoptotic disease-effector cells such as apoptotic tumor-effector cells and/or disease-associated antigens such as tumor-associated antigens.

Activated monocytes as they are obtainable by methods of the first and second aspect and their embodiments can be used to treat patients suffering from a cancer. While they probably will be suitable for treatment of cancer patients undergoing chemotherapy and/or radiation therapy, they may be particularly suitable for patients which are not under chemotherapy and/or gamma-irradiation therapy yet. They may also be particular suitable for preventive vaccination against certain cancers. Activated monocytes as their obtainable by methods of the third aspect and its embodiments can also be used to treat patients suffering from cancer. While they are assumed to suitable for treatment of cancer patients not undergoing chemotherapy and/or gamma-irradiation therapy yet, they may be particularly suitable for patients under such treatments. However, in one embodiment globally activated monocytes as they are obtainable according to the third aspect are not used in patients who are treated with therapeutic antibodies.

The term "for use in treatment of' is considered to be equivalent to the use of such immuno-stimulatory autologous antigen-presenting cells in the manufacture of a medicament for preventive or curative treatment of patients suffering from disease being mediated by the disease-effector cells or disease-associated antigens, for example preventive or curative treatment of tumors, preferably solid tumors.

The term "for use in treatment of' is also considered to be equivalent to methods of curative or preventive treatment of a disease being mediated by the disease-effector cells or disease-associated antigens, for example preventive or curative treatment of tumors, preferably solid tumors, by administering to a patient in need thereof immuno-stimulatory autologous antigen-presenting cells such as dendritic cells as they are obtainable by the methods of the first and second aspect and their embodiments.

For the purposes of the present disclosure the term "curative treatment" refers to the curative alleviation of a disease while the term "preventive treatment" refers to preventive prophylaxis. It is to be understood that both terms do not imply a complete remission or prevention of the respective disease but rather that there is an improvement compared to a situation where no immuno-stimulatory autologous APCs are administered for either curative or preventive purposes.

As used herein, the term "disease-effector cells" or "disease-associated antigens" refers to cells and antigens that are central to the causation of a disease state in a subject. For example immuno-stimulatory antigen-presenting cells such as dendritic cells may be loaded with antigens, which are known being expressed in cancerous tissue. To this end, such antigens may be expressed in immuno-stimulatory dendritic cells such that they are displayed on the MHC class 1 and MHC class 2 molecules. Isolated disease-associated antigens or isolate tumor-associated antigens refer to antigens which have been obtained e.g. from disease-effector cells such as tumor cells by purification, recombinant expression or synthetic manufacture. One may for example analyze a patient's tumor sample, identify the most highly expressed tumor-associated antigens and then prepare a mixture of these antigens by e.g. recombinant expression or synthetic chemistry.

In a preferred embodiment, the isolated disease-associated antigen comprises a synthetic or a recombinant peptide.

By loading immuno-stimulatory autologous antigen-presenting cells such as dendritic cells with such antigens, subjects may be vaccinated against the later occurrence of e.g. a cancer or an infection thereby enabling preventive treatment. If an antigen is used, which is already expressed by cancerous tissue obtained from the subject, from whom the extracorporeal amount of blood was taken to generate immuno-stimulatory antigen-presenting cells, the antigen-loaded, reintroduced immuno-stimulatory antigen-presenting cells such as dendritic cells may launch an immune response against the cancer thereby allowing for curative treatment.

In certain circumstances, these disease-effector cells and preferably tumor cells are disease-causing cells which may be circulating in the bloodstream, thereby making them readily accessible to extracorporeal manipulations and treatments. Examples of such disease-causing cells include e.g. malignant T-cells, malignant B-cells, and virally or bacterially infected white or red blood cells which may harbor or express microbial (e.g. viral, bacterial, fungal, mycobacterial, protozoal) peptides or proteins or other pathogen-associated molecules. Exemplary disease categories giving rise to disease-causing cells include lymphoproliferative disorders such as leukemia, lymphoma, as well as infections including malaria, human-immunodeficiency virus (HIV), Epstein-Barr virus (EBV), cytomegalovirus (CMV), hepatitis B virus (HBV), hepatitis C virus (HCV), Lyme disease, leprosy, tuberculosis, and other blood borne pathogens.

Other tumor cells and TAAs cells include those isolated from surgically excised specimens from solid tumors, such as lung, glioma, breast, colon, prostate, head and neck cancer, brain, connective tissue, kidney cancer or skin cancers such as melanoma. Such cells may be obtained from the solid tumor itself. These cells can be manipulated extracorporeal, i.e. outside the human or animal body, to induce apoptosis thereof and shedding of TAAs. Alternatively or in addition, in some instances, it has been shown that the circulating blood of patients with solid tumors can contain malignant cells that have broken off from the tumors and entered the circulation. These circulating tumor cells of solid tumors can provide an easily accessible source of cancer cells and thus TAAs, which may be isolated, rendered apoptotic and engulfed by the dendritic cells in accordance with the method described and claimed herein.

While the methods disclosed herein provide for treatment of solid tumors, they may of course also allow for treatment of cancers such as CTCLs or other lymphomas. Using the methods disclosed herein may allow for an improved treatment of such tumors given that for the reasons set out above a treatment cycle may require obtaining only once monocytes from the patient other than for the two day treatment cycle during ECP.

TAAs may also be obtained from such cancer-causing cells by inducing apoptosis by cytotoxic agents or PUVA. Cytotoxic agents include taxanes including docetaxel and paclitaxel, anthracyclines, cisplatin, carboplatin, 5-fluoro-uracil, gemcitabine, capecitabin, navelbine or zoledronate. One may, of course, also use an isolated or even recombinantly expressed TAA or combinations of such isolated or recombinantly expressed TAAs which is or are known to be identified and possibly effective TAAs.

In order to avoid e.g. protein degradation of delivered TAAs and inefficient processing of soluble TAAs by antigen-presenting cells such as dendritic cells, it is contemplated to enhance formation of autologous TAA-displaying antigen-presenting cells by encapsulation of TAAs in polymeric nanoparticles (NPs), which may be made from biodegradable polymers such as polylactic acid (Waeckerle-Men et al., Adv Drug Deliv Rev (2005), 57:475-82). Other means for increasing delivery and processing of TAAs to and by APCs are known to the skilled person and also contemplated by the present disclosure.

Thus the description contemplates to use encapsulation of antigens in polymeric nanoparticles to optionally further enhance formation of immuno-stimulatory autologous APCs such as dendritic cells.

In another preferred embodiment of the present invention, the antigen-presenting cells displaying disease-associated antigens are combined with an extracorporeal quantity of lymphocytes. The extracorporeal quantity of lymphocytes may comprise natural killer cells and/or T-lymphocytes (also called "T-cells"). The lymphocytes may be obtained by isolating the cells from a mammalian subject's sample, such as a blood sample or a tissue sample, and optionally by further cultivating the cells in vitro. After combining the antigen-presenting cells displaying disease-associated antigens with the extracorporeal quantity of lymphocytes, the cells may then be cultivated in vitro for at least 12 hours, at least 1 day, at least 3 days, at least 7 days or at least 14 days. The antigen-presenting cells displaying disease-associated antigens will then activate the lymphocytes. After the incubation, the cells or a fraction thereof may be administered to the patient.

In one embodiment, the lymphocytes are genetically modified lymphocytes, wherein the cells are genetically modified by methods known to the person skilled in the art.

In a preferred embodiment, the lymphocytes comprise T-lymphocytes, wherein the T-lymphocytes preferably comprise CD8⁺ T-lymphocytes. The antigen-presenting cells displaying disease-associated antigens will then activate the T-lymphocytes in an antigen-specific manner.

In a preferred embodiment of the present invention, the activated lymphocytes are for use in the treatment of solid tumors as described above. They may of course also be used for treatment of cancers such as CTCLs or other lymphomas.

The invention is now described with respect to some specific examples which, however, are for illustrative purposes and not to be construed in a limiting manner.

### Experiments

### Experiment 1

### Materials and Methods

### Generation of melanoma mouse model

The known YUMM 1.7 melanoma cell line (Theodosakis, N et al., Mol Cancer Ther. Published OnlineFirst May 6, 2015; doi:10.1158/1535-7163.MCT-15-0080) was used for generating melanoma tumors in male C57BL/6 mice. 10⁵ YUMM 1.7 cells in PBS were injected subcutaneously in nine 4 week old male C57BL/6 mice under the right flank to induce tumor formation.

Mice were grown for approximately 11-13 days to establish small tumors at about 10 mm³. The mice were then divided into two cohorts. One cohort (four mice) was designated as the treatment group (Group 1) and the second cohort (five mice) was designated as the PBS control group (Group 2).

After days 11-13, each treatment for Group 1 was started by bleeding the mice and taking 200 µl of entire blood per mouse. Blood was spun through a Ficoll gradient to remove red blood cells and to obtain peripheral blood mononuclear cells (PBMC) at an amount of 8.33^{∗}10⁸ cells/ml. In parallel, the same number of YUMM 1.7 cells was suspended in PBS and subjected to 8-MOP and UVA treatment (4 J/cm² and 100ng/ml 8-MOP) by passing the Yumm 1.7 cells through a flow chamber as depicted in Figure 1. The flow rate was 0.1 ml/min.

8-MOP/UVA-treated Yumm 1.7 cells were then mixed with PBMCs and passed through the same flow chamber. The flow rate was 0.1 ml/min and subjected to 8-MOP and UVA treatment (2 J/cm² and 100ng/ml 8-MOP).

Cells were spun down, resuspended in mice's serum (100 µl per mouse) and intravenously injected back inside the retrorbital sinus of the mice.

For Group 2, the same procedure was performed except that PBMC were replaced by PBS buffer. This procedure was repeated twice a week over the next three weeks (overall six treatments). Subsequently tumor volume was determined by cell counting.

### Results

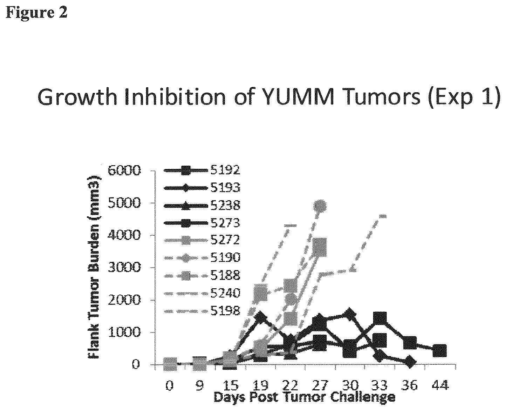

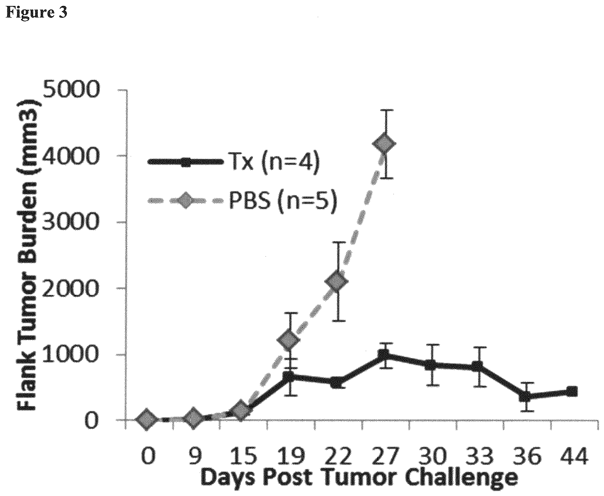

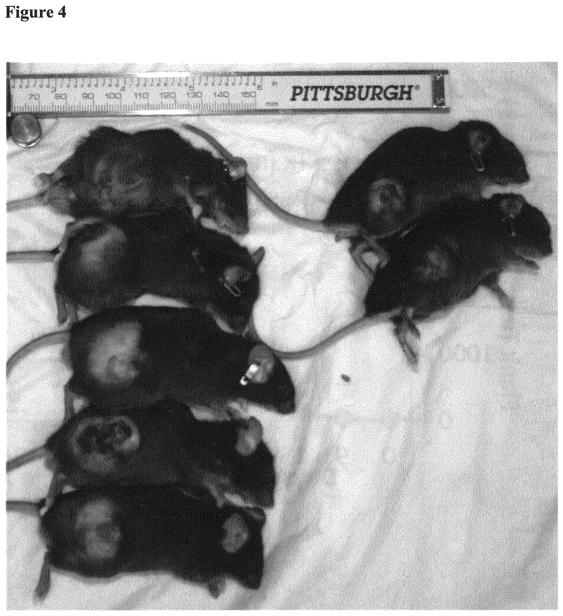

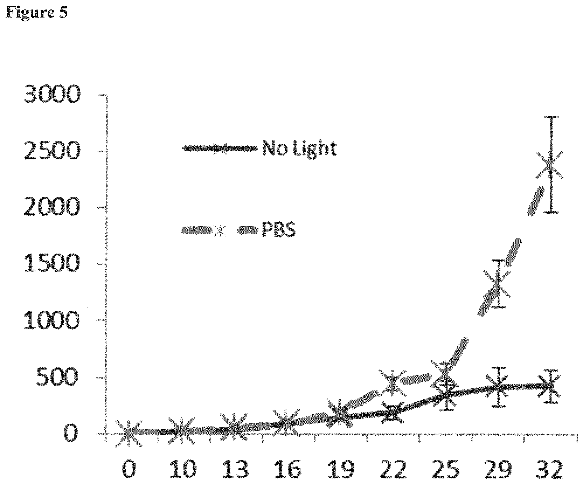

The results for the individual mice are shown in Figure 2. The combined results are shown in Figure 3. Figure 4 depicts some of the treated mice. The results are shown in Figure 5. A clear reduction of tumor volume is observed for Group 1, but not for Group 2.

### Experiment 2

### Materials and Methods

YUMM 1.7 cells were subcutaneously injected to generate tumors as described in Experiment 1.

Mice were grown for approximately 11-13 days to establish small tumors at about 10 mm³. The mice were then divided into two cohorts. One cohort (five mice) was designated as the treatment group (Group 1) and the second cohort (five mice) was designated as the PBS control group (Group 2).

After days 11-13, each treatment for Group 1 was started by bleeding the mice and taking 200 µl of entire blood per mouse. Blood was spun through a Ficoll gradient to remove red blood cells and to obtain PBMCs at an amount of 8.3^{∗}10⁸ cells/ml. In parallel, the same number of YUMM 1.7 cells was suspended in PBS and subjected to 8-MOP and UVA treatment (4 J/cm² and 100ng/ml 8-MOP) by passing the Yumm 1.7 cells through a flow chamber as depicted in Figure 1. The flow rate was 0.1 ml/min.

8-MOP/UVA-treated Yumm 1.7 cells were then mixed with PBMCs and passed through the same flow chamber but other than in Experiment 1 without applying 8-MOP and UVA. The PBMCs were thus not subject to any apoptotic challenge.

Cells were spun down, resuspended in mice's serum (100 µl per mouse) and intravenously injected back inside the retrorbital sinus of the mice.

For Group 2, the same procedure was performed except that PBMC were replaced by PBS buffer. This procedure was repeated twice a week over the next three weeks (overall six treatments). Subsequently tumor volume was determined by cell counting.

### Results

The results are shown in Figure 5. A clear reduction of tumor volume is observed for Group 1, but not for Group 2.

### Experiment 3

### Materials and Methods

YUMM 1.7 cells were subcutaneously injected to generate tumors as described in Experiment 1.

Mice were grown for approximately 11-13 days to establish small tumors at about 10 mm³. The mice were then divided into four cohorts. One cohort (five mice) was designated as the PBS control group (Group 1), three cohorts (Groups 2 to 4, each five mice) were treatment groups.

After days 11-13, each treatment for Group 2 was started by bleeding the mice and taking 200 µl of entire blood per mouse. Blood was spun through a Ficoll gradient to remove red blood cells and to obtain PBMCs at an amount of 8.3^{∗}10⁸ cells/ml. In parallel, the same number of YUMM 1.7 cells was suspended in PBS and subjected to 8-MOP and UVA treatment (4 J/cm² and 100ng/ml 8-MOP) by passing the Yumm 1.7 cells through a flow chamber as depicted in Figure 1. The flow rate was 0.1 ml/min.

Yumm 1.7 cells were spun down, resuspended in mice's serum (100 µl per mouse) and intravenously injected back inside the retrorbital sinus of the mice without PBMCs (Yumm alone). This procedure was repeated twice a week over the next three weeks (overall six treatments). Subsequently tumor volume was determined by cell counting.

For Group 1, the same treatment procedure was performed except that pure PBS (without Yumm cells or PBMCs) was injected into mice.

For Group 3, PBMCs were obtained as described for Group 1. PBMCs were resuspended in PBS and passed through the flow chamber of Figure 1 at a flow rate of 0.1 ml/min without 8-MOP/UVA treatment. Flow-chamber passaged PBMCs were spun down, resuspended in mice's serum (100 µl per mouse) and intravenously injected without Yumm cells back inside the retrorbital sinus of the mice (PBMC, PP w/o YUMM). This procedure was repeated twice a week over the next three weeks (overall six treatments). Subsequently tumor volume was determined by cell counting.

For Group 4, PBMCs were obtained as for Group 3 and passed through the flow chamber of Figure 1 at a flow rate of 0.1 ml/min without 8-MOP/UVA treatment. In parallel, YUMM 1.7 cells were suspended in PBS and subjected to 8-MOP and UVA treatment (4 J/cm² and 100ng/ml 8-MOP) by passing the Yumm 1.7 cells through the flow chamber as depicted in Figure 1 at a flow rate was 0.1 ml/min.

8-MOP/UVA-treated Yumm 1.7 cells and PBMCs were then co-incubated overnight at 37° C and 5% CO₂ in RPMI medium supplemented with 15% mouse plasma. Cells were spun down, resuspended in mice's serum (100 µl per mouse) and intravenously injected back inside the retrorbital sinus of the mice (O/N YUMM^{UVA} PP^{noUVA}). This procedure was repeated twice a week over the next three weeks (overall six treatments). Subsequently tumor volume was determined by cell counting.

### Results

The results are shown in Figure 6. A clear reduction of tumor volume is observed for Group 2 and Group 3 vs the control Group 1. Tumor reduction is most advanced with Group 4.

### Experiment 4

### Materials and Methods

YUMM 1.7 cells were subcutaneously injected to generate tumors as described in Experiment 1.

Mice were grown for approximately as described in Example 3 for 11-13 days to establish small tumors at about 10 mm³. The mice were then divided into three cohorts. One cohort was designated as the PBS control group and obtained as described for Group 1 in Experiment 3. The other two cohorts were the same as described for Group 4 in Experiment 3 except that flow chamber passed PBMCs and PUVA-treated Yumm 1.7 cells were either co-incubated overnight or not.

### Results

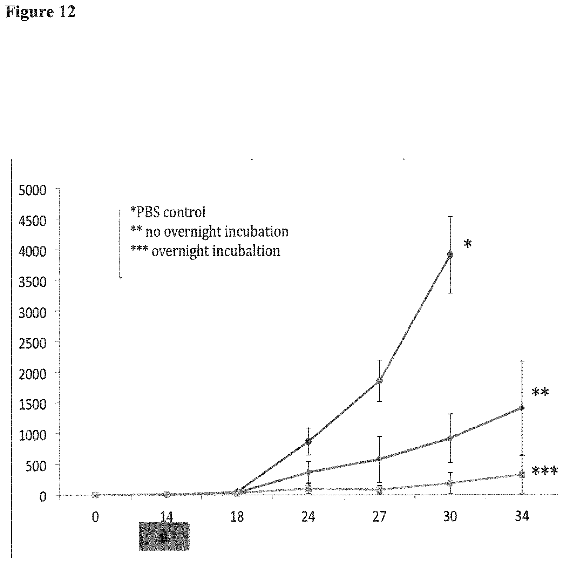

The results are shown in Figure 12.

### Experiment 5

### Determining expression of molecular markers and FSC/SSC complexity after passing monocytes through flow chamber

### Materials and Methods

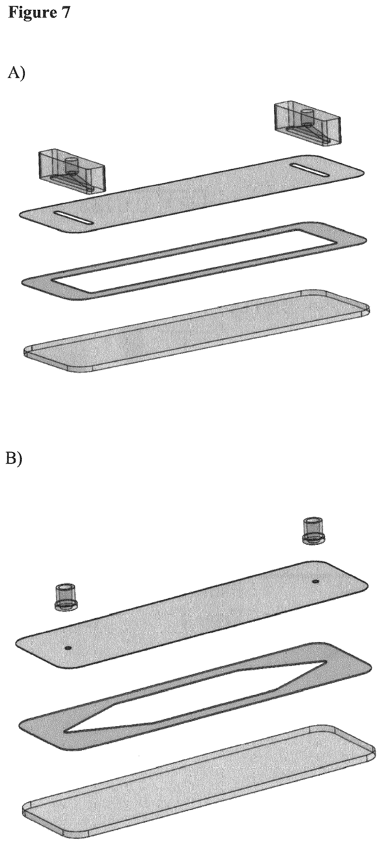

Monocytes were passed through a device as depicted in Fig. 7. In brief, a blood sample was spun at low speed through a Ficoll gradient to obtain e.g. 8 ml of sample with a concentration of peripheral blood mononuclear cells (PBMC) of e.g. 10¹⁰ cells/ml. The chamber was pre-coated with platelets. The sample was passed through the chamber at about 0.028 Pa. The chamber and then washed with about 3 ml RPMI at 0.028 Pa. A second wash with 30-55 ml RPMI was performed at about 1.2 Pa. The collected activated monocytes were combined, incubated for a day and used for further analysis (PP D1 PBMC). As a control PBMCs were not passed through the device and incubated for a day (D1 PBMC). As another control immature fast DC were obtained by directly cultivating PBMC in the presence of GM-CSF and IL-4 (immature Fast DC). Further, PBMC were analyzed directly after harvest through a Ficoll gradient (Fresh (Ficoll) PBMC).

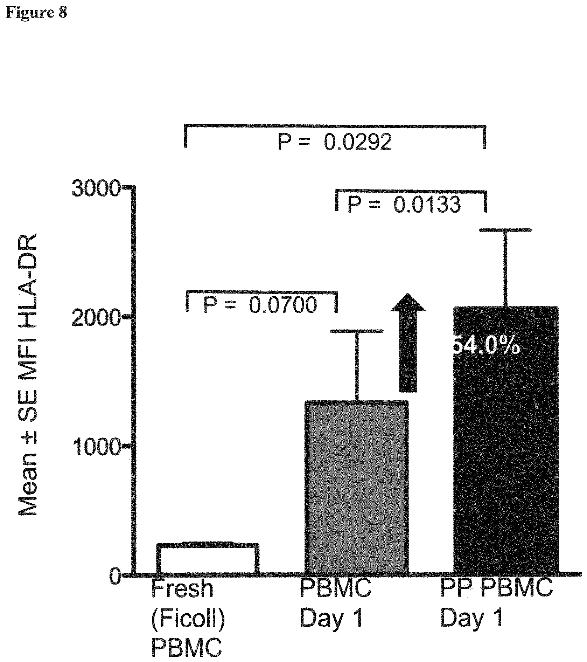

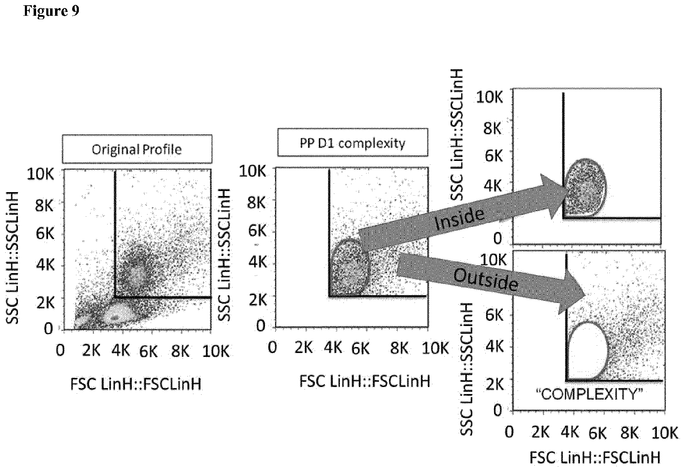

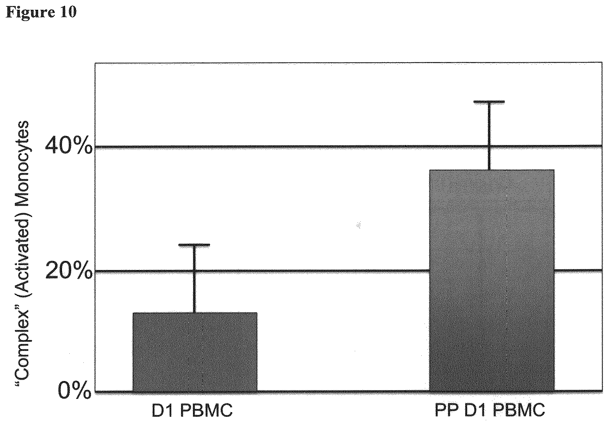

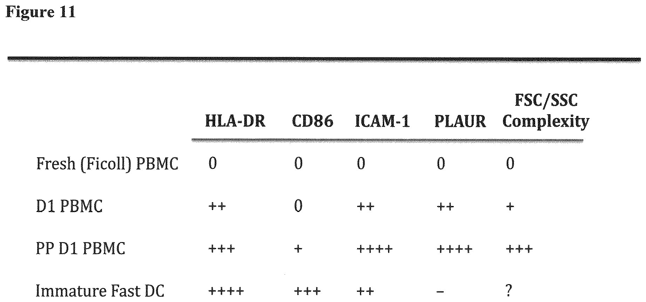

The cells and controls were then analyzed for expression of HLA-DR, CD86, ICAM-1, and PLAUR. They were further analyzed for FSC/SSC complexity. The results are depicted for HLA-DR in Figure 8 and for FSC/SSC complexity in Figure 9 and 10. A summary is shown in Figure 11.

### Results

The results show that cells subjected to centrifugation through a Ficoll gradient already seem to experience enough physical forces to start differentiating as becomes apparent from incubating these cells for one day (D1 PBMC). However, activation and differentiation is more pronounced upon plate passage through the device (PP D1 PDMC). The dendritic cells obtained by methods in accordance with the invention in the absence of e.g. 8-MOP and UV-A moreover have a more complex and distinct pattern than immature Fast DC obtained with cytokine cocktails.

### Example 6

### Materials and Methods

1×10⁵ YUMM1.7 cells were inoculated in 100 µL PBS, subcutaneously, into the right flank of syngeneic C57BL/6 mice. At day 7-10 after tumor inoculation (palpable tumor in all animals), 200 µL of peripheral blood was collected from the cheek of each mouse into heparin, and combined. The PBMC were separated by layering the blood over an equal volume of Lympholyte M, followed by treatment of PBMC with red blood cell lysis buffer. 2.5×10⁶ YUMM1.7 cells were incubated at 37°C for 20 min with 100 ng/mL 8-MOP, and exposed to 4 J/cm² UVA irradiation. Irradiated YUMM1.7 cells were combined 1:1 with 2.5×10⁶ isolated PBMCs in FBS in a total volume of 600 µL, incubated in the transimmunisation (TI) plate for 1hr at 37° C, and then passed through the TI plate at a flow rate of 0.09 mL/min. The plate was then flushed with 600 µL of FBS at 0.49 mL/min and the flow-through added to the plate-passed cells. Plate-passed PBMCs and YUMM1.7 cells were then either incubated at 37°C for 20 min with 100 ng/mL 8-MOP, and exposed to 4 J/cm² UVA irradiation ("apoptotic agent (+)") or the cells were not exposed to 8-MOP/UVA ("apoptotic agent (-)"). The cells were cultured overnight at 37 °C in clear RPMI medium supplemented with L-glutamine, penicillin/streptomycin and 15% FCS. After overnight culture, the treated cells were collected, resuspended in autologous plasma at 1×10⁷ cells/mL, and 100 mL injected into the retro-orbital plexus of experimental tumor-bearing mice, while control animals received 100 µL of autologous plasma or PBS ("PBS"). The blood was collected on Mondays and Thursdays, and cells re-injected in Tuesdays and Wednesdays, for a total of 3 weeks/6 treatments for each mouse. Tumor volumes of experimental and control animals were monitored throughout the experiment by caliper measurements of tumor dimensions.

The study was repeated twice.

### Results

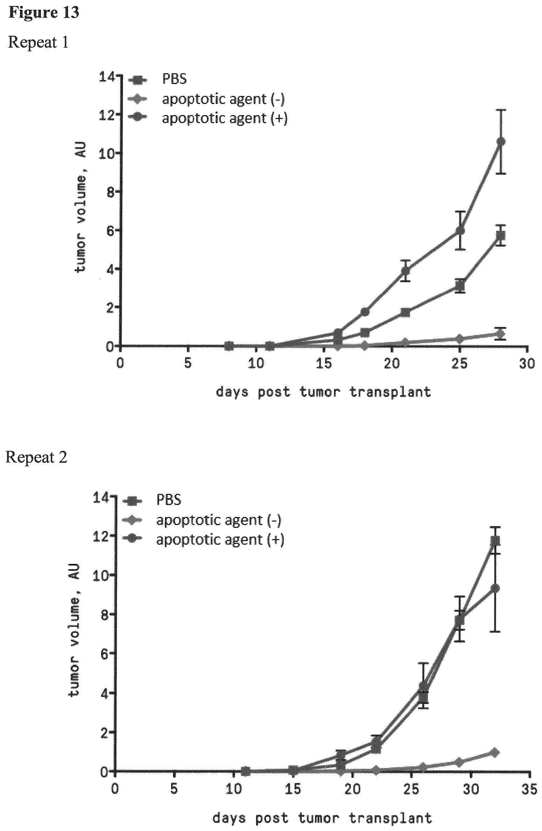

The results are shown in Figure 13. Without exposure to 8-MOP/UVA, the plate-passed PBMCs and YUMM1.7 cells significantly reduce the tumor volumes. The tumor-reducing effect is neutralized (repeat 2) or the tumor-volume even increases compared to the negative control (repeat 1) if the animals were treated with the combination of plate-passed PBMCs and YUMM1.7 cells exposed to 8-MOP/UVA.

### Example 7

### Materials and Methods

1×10⁵ YUMM1.7 cells were inoculated in 100 µL PBS, subcutaneously, into the right flank of syngeneic C57BL/6 mice. At day 7-10 after tumor inoculation (palpable tumor in all animals), 200 µL of peripheral blood was collected from the cheek of each mouse into heparin, and combined. The PBMC were separated by layering the blood over an equal volume of Lympholyte M, followed by treatment of PBMC with red blood cell lysis buffer. 2.5×10⁶ YUMM1.7 cells were incubated at 37°C for 20 min with 100 ng/mL 8-MOP, and exposed to 4 J/cm² UVA irradiation. Irradiated YUMM1.7 cells were combined 1:1 with 2.5×10⁶ isolated PBMCs in FBS in a total volume of 600 µL, incubated in the TI plate for 1 hr at 37° C, and then passed through the TI plate at a flow rate of 0.09 mL/min. The plate was then flushed with 600 µL of FBS at 0.49 mL/min and the flow-through added to the plate-passed cells.

In parallel, additional PBMC cells were isolated from tumor-bearing but untreated mice controls and incubated at 37°C for 20 min with 100 ng/mL 8-MOP, and exposed to 4 J/cm² UVA irradiation ("apoptotic agent-treated PBMCs")

Plate-passed PBMCs and YUMM1.7 cells were then either combined with 2.5×10⁶ apoptotic agent-treated PBMCs (" apoptotic agent-treated PBMCs (+)") or not (" apoptotic agent-treated PBMCs (-)"). The cells were then cultured overnight at 37 °C in clear RPMI medium supplemented with L-glutamine, penicillin/streptomycin and 15% FCS. After overnight culture, the treated cells were collected, resuspended in autologous plasma at 1×10⁷ cells/mL, and 100 mL injected into the retro-orbital plexus of experimental tumor-bearing mice, while control animals received 100 µL of autologous plasma or PBS ("PBS"). The blood was collected on Mondays and Thursdays, and cells re-injected in Tuesdays and Wednesdays, for a total of 3 weeks/6 treatments for each mouse. Tumor volumes of experimental and control animals were monitored throughout the experiment by caliper measurements of tumor dimensions.

### Results

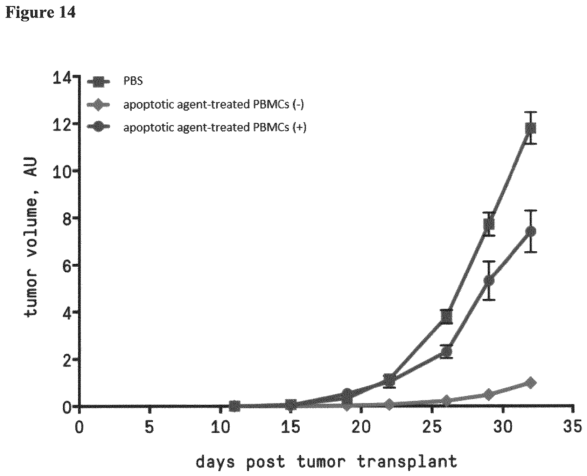

The results are shown in Figure 14. In the absence of apoptotic agent-treated PBMCs, the plate-passed PBMCs and YUMM1.7 cells significantly reduce the tumor volumes. The tumor-reducing effect is neutralized if the animals were treated with a mixture of the plate-passed PBMCs and YUMM1.7 cells with apoptotic agent-treated PBMCs prior to incubation.

References:
1. Matsushita H, Vesely MD, Koboldt DC, Rickert CG, Uppaluri R, et al. Cancer exome analysis reveals a T-cell-dependent mechanism of cancer immunoediting. Nature 2012;482:400-404.
2. Tran E, Turcotte S, Gros A, et al. Cancer immunotherapy based on mutation-specific CD4+ T cells in a patient with epithelial cancer. Science 2014;344:641-645
3. van Rooij N, van Buuren MM, Philips D, et al. Tumor exome analysis reveals neoantigen-specific T-cell reactivity in an ipilimumab-responsive melanoma. J Clin Oncol 2013;31:e439-e442.
4. Kantoff PW, Higano CS, Shore ND, Berger ER, Small EJ, et al. Sipuleucel-T immunotherapy for castration-resistant prostate cancer. N Engl J Med 2010;363:411-422.
5. Kalos M, Levine BL, Porter DL, Katz S, Grupp SA, et al. T cells with chimeric antigen receptors have potent antitumor effects and can establish memory in patients with advanced leukemia. Sci. Transl. Med 2011;3:95ra73.
6. Edelson RL, Berger C, Gasparro F, Jegasothy B, Heald P, et al., Treatment of cutaneous T-cell lymphoma by extracorporeal photochemotherapy. Preliminary results. N Eng J Med 1987;316(6):297-303.
7. Heald P, Rok A, Perez M, Wintroub B, Knobler R, et al. Treatment of erythrodermic cutaneous Tcell lymphoma with extracorporeal photochemotherapy. J Amer Acad Dermatol 1992;17:427-33.
8. Girardi M, Knobler R, Edelson RL. Selective immmunotherapy through extracorporeal photochemotherapy: yesterday, today, and tomorrow. Hematol Oncol Clin N Am 2003;17:13911403.
9. Greinix HT, Volc-Platzer B, Rabtsch W, Gmeinhart B, Guevara-Pineda C, et al. Successful use of extracorporeal photochemotherapy in the treatment of severe acute and chronic graft-versus-host disease. Blood 1998;92:3098-104.
10. Rook AH, Freundlich B, Jegasothy BV, Perez MI, Barr WG, Jimenez SA, et al. Treatment of systemic sclerosis with extracorporeal photochemotherapy - results of a multicenter trial. Arch Dermatol. 1992;128:337-e46.
11. Di Spaltro FX, Cottrill C, Cahill C, Degnan E, Mulford GJ, et al. Extracorporeal photochemotherapy in progressive systemic sclerosis. Int J Dermatol. 1993;32:1-5.
12. Wolfe JT, Lessin SR, Singh AH, Rook AH. Review of immunomodulation by photopheresis: treatment of cutaneous T-cell lymphoma, autoimmune disease and allograft rejection. Artif Organs. 1994;18(12): 888-97.
13. Gonzalez AI, Berger CL, Remington J, Girardi M, Tigelaar RE, Edelson RL. Integrin-driven monocyte to dendritic cell conversion in modified extracorporeal photochemotherapy. Clin and Exp Immunology. 2013;175:449-457.
14. Berger C, Hoffmann K, Vasquez JG, Mane S, Lewis J, et al., Rapid generation of maturationally synchronized human dendritic cells: contribution to the clinical efficacy of extracorporeal photochemotherapy. Blood. 2010. 116(23):4838-47.
15. Futterleib JS, Feng H, Tigelaar RE, Choi J, Edelson RL. Activation of GILZ gene by photoactivated 8-methoxypsoralen: potential role of immunoregulatory dendritic cells in extracorporeal photochemotherapy. Transfus Apher Sci. 2014;50(3):379-87.
16. de Saint-Vis B, Vincent J, Vandenabeele S, Vanbervliet B, Pin JJ, Ait-Yahia S, et al. A novel lysosome-associated membrane glycoprotein, DC-LAMP, induced upon DC maturation, is transiently expressed in MHC class II compartment. Immunity. 1998;9:325-36.
17. Lamioni A, Parisi F, Isacchi G, Giorda E, Di CeSare S, et al., The immunological effects of extracorporeal photopheresis unraveled: Induction of tolerogenic dendritic cells in vitro and regulatory T cells in vivo. Transplantation. 2005. 79(7):846-50.
18. Berger CL, Xu AL, Hanlon D, Girardi M, Edelson R. Induction of human tumor-loaded dendritic cells. Int J Cancer. 2001;91:438- 47.
19. Banchereau J, Briere F, Caux C, Davoust J, Lebecque S, et al. Immunobiology of dendritic cells. Annu Rev Immunol. 2000;18(1):767-811.
20. Sllusto F, Geginat J, Lanzavecchia A. Central and memory effector memory T cell subsets: function, generation, and maintenance. Annu Rev Immunol. 2004;22(1):745-63.
21. Alanio C, Lemaitre F, Law HK, Hasan M, Albert ML. Enumeration of human antigen-specific naive CD8+ T cells reveals conserved precursor frequencies. Blood. 2010;115(18):3718-3725.
22. Coulie PG, Brichard V, Van Pel A, Wolfel T, Schneider J, et al. A new gene coding for a differentiation antigen recognized by autologous cytolytic T lymphocytes on HLA-A2 melanomas. J Exp Med 1994;180:35-42.
23. Faure FA, Mantegazza C, Sadaka C, Sedlik F, Amigorena S. Long-lasting cross-presentation of tumor antigen in human DC. Eur. J. Immunol. 2009;39: 380-390.
24. Bioley G, Jandus C, Tuyaerts S, Rimoldi D, Kwok WW, et al. MelanA/MART-1-specific CD4 T cells in melanoma patients: identification of new epitopes and ex vivo visualization of specific T cells by MHC class II tetramers. J Immunol 2006;177:6769-6779.
25. Griffoen AW, Rijkers GT, Keij J, Zegers BJ. Measurement of cytoplasmic calcium in lymphocytes during flow cytometry. J Immunol Methods 1989;120(1):23-27.
26. Kvistborg P, Boegh M, Pederson AW, Claesson MH, Zocca MB. Fast generation of dendritic cells. Cell Immunol 2009;260(l):56-62.
27. Yewdall AW, Drutman SB, Jinwala F, Bahjat KS, Bhardwaj N. CD8+ T cell priming by dendritic cell vaccines requires antigen transfer to endogenous antigen presenting cells. PLOS One 2010;5(6):1-10.
28. Johnson LA, Heemskerk B, Powell DJ Jr, Cohen CJ, Morgan RA, et al. Gene transfer of tumorreactive TCR confers both high avidity and tumor reactivity to nonreactive peripheral blood mononuclear cells and tumor-infiltrating lymphocytes. J Immunol. 2006;177(9):6548-59.
29. Stipdonk MJB, Lemmens EE, Schoenberger SP. Naive CTLs require a single, brief period of antigenic stimulation for clonal expansion and differentiation. Nature Immunol. 2001;2(5):423-29.
30. Irving M, Zoete V, Hebeisen M, Schmid D, Baumgartner P, et al. Interplay between T Cell Receptor Binding Kinetics and the Level of Cognate Peptide Presented by Major Histocompatibility Complexes Governs CD8 + T Cell Responsiveness. J Biol Chem 2012;287(27):23068-78.
31. Wolfl M, Greenberg PD. Antigen-specific activation and cytokine-facilitated expansion of naive human CD8+ T cells. Nature Protocols 2014;9(4):950-66.
32. Beeson C, Rabinowitz J, Tate K, Gutgemann I, Chien Y, et al. Early biochemical signals arise from low affinity TCR-ligand reactions at the cell-cell interface. J Exp Med. 1996;184(2):777-82.
33. Stern E, Steenbock ER, Reed MA, Fahmy TM. Label-free electronic detection of the antigen specific T-cell immune response. Nanoletters. 2008;8(10):3310-14.
34. Durazzo TS, Tigelaar RE, Filler R, Hayday A, Girardi M, Edelson RL. Induction of monocyte-to-dendritic cell maturation by extracorporeal photochemotherapy: initiation via direct platelet signaling. Transf and Apheresis Sc. 2014;50(3):370-78.

## Claims

1. Method for obtaining activated lymphocytes, comprising at least the steps of
a. providing antigen-presenting cells displaying disease-associated antigens, wherein the antigen-presenting cells are obtained by the following steps of
i. providing an extracorporeal sample of mammalian disease-effector cells and subjecting said disease-effector cells to apoptotic agents to release disease-associated antigens or providing isolated disease-associated antigens,
ii. providing an extracorporeal quantity of a mammalian subject's blood sample comprising monocytes separately from step i),
iii. activating said monocytes to induce their differentiation into immuno-stimulatory autologous APCs in the absence of apoptotic agents separately from step i),
iv. combining said immuno-stimulatory autologous APCs of step iii) with said apoptotic disease-effector cells or isolated disease-associated antigens of step i); wherein alternatively, step iv. can be performed prior to performing step iii. such that said apoptotic disease effector cells or isolated disease-associated antigens of step i) are combined with said monocytes of step ii), followed by activation of said monocytes in the mixture to induce their differentiation into immuno-stimulatory autologous APCs in the absence of apoptotic agents.
b. providing an extracorporeal quantity comprising CD8⁺ T-lymphocytes or genetically modified T-lymphocytes;
c. combining said antigen-presenting cells displaying disease-associated antigens of step a. with said lymphocytes of step b); and
d. incubating the mixture of step c), wherein the antigen-presenting cells displaying disease-associated antigens will activate the lymphocytes to proliferate.

2. Method according to claim 1, wherein the incubation of step d) is for at least 12 hours, at least 1 day, at least 3 days, at least 7 days or at least 14 days.

3. Method according to claim 1 or 2, wherein said lymphocytes of step b) are obtained from a mammalian subject's sample, e.g. a blood or tissue sample.

4. Method according to any of the preceding claims, wherein said genetically modified lymphocytes are CAR-T lymphocytes.

5. Method according to claim 1, wherein the apoptotic agent is selected from the group comprising taxanes including docetaxel and paclitaxel, anthracyclines, cisplatin, carboplatin, 5-fluoro-uracil, gemcitabine, capecitabin, navelbine or zoledronate, and/or the combination of 8-MOP and UVA.

6. Method according to claim 1 or 4, wherein said monocytes are activated and induced to differentiate into immuno-stimulatory autologous antigen-presenting cells by subjecting said monocytes to a shear force in a flow chamber of a device.

7. Method according to claims 1, 4 or 5, wherein said monocytes are activated and induced to differentiate into immuno-stimulatory autologous antigen-presenting cells through interaction with platelets and/or plasma components in the absence of apoptotic agents.

8. Method according to claims 1, 4, 5 or 6, wherein said disease-effector cells are cells taken from a tumor and wherein said released disease-associated antigens are released tumor-associated antigens.

9. Method of claim 7, wherein said tumor cells are obtained from or shed from a solid tumor.

10. Method of claim 8, wherein said solid tumor is selected from the group comprising lung cancer, breast cancer, pancreatic cancer, colon cancer, prostate cancer, head and neck cancer, glioma, brain cancer, ovarian, muscle, connective tissue, kidney cancer or skin cancers such as melanoma.

## Patentansprüche

1. Verfahren zur Gewinnung von aktivierten Lymphozyten, das mindestens die folgenden Schritte umfasst
a. Bereitstellung von Antigen-präsentierenden Zellen, die krankheitsassoziierte Antigene aufweisen, wobei die Antigen-präsentierenden Zellen durch die folgenden Schritte erhalten werden
i. Bereitstellen einer extrakorporalen Probe von Säugetier-Krankheitseffektorzellen und Aussetzen der Krankheitseffektorzellen gegenüber apoptotischen Mitteln, um krankheitsassoziierte Antigene freizusetzen, oder Bereitstellen isolierter krankheitsassoziierter Antigene,
ii. Bereitstellung einer extrakorporalen Menge einer Blutprobe eines Säugetieres, die Monozyten enthält, getrennt von Schritt i),
iii. Aktivierung der Monozyten, um ihre Differenzierung in immunstimulierende autologe APCs in Abwesenheit von apoptotischen Mitteln getrennt von Schritt i) zu induzieren,
iv. Kombinieren der immunstimulierenden autologen APCs von Schritt iii) mit den apoptotischen Krankheitseffektorzellen oder isolierten krankheitsassoziierten Antigenen von Schritt i); wobei alternativ Schritt iv. vor der Durchführung von Schritt iii. durchgeführt werden kann, so dass die apoptotischen Krankheitseffektorzellen oder isolierte krankheitsassoziierte Antigene von Schritt i) mit den Monozyten von Schritt ii) kombiniert werden, gefolgt von einer Aktivierung der Monozyten in der Mischung, um ihre Differenzierung in immunstimulierende autologe APCs in Abwesenheit von apoptotischen Mitteln zu induzieren.
b. Bereitstellung einer extrakorporalen Menge, die CD8⁺ T-Lymphozyten oder genetisch veränderte T-Lymphozyten enthält;
c. Kombinieren der Antigen-präsentierenden Zellen, die krankheitsassoziierte Antigene aufweisen, aus Schritt a) mit den Lymphozyten aus Schritt b); und
d. Inkubation der Mischung aus Schritt c), wobei die Antigen-präsentierenden Zellen, die krankheitsassoziierte Antigene aufweisen, die Lymphozyten zur Proliferation aktivieren.

2. Verfahren nach Anspruch 1, wobei die Inkubation von Schritt d) mindestens 12 Stunden, mindestens 1 Tag, mindestens 3 Tage, mindestens 7 Tage oder mindestens 14 Tage lang dauert.

3. Verfahren nach Anspruch 1 oder 2, wobei die Lymphozyten aus Schritt b) aus einer Säugetierprobe, z.B. einer Blut- oder Gewebeprobe, gewonnen werden.

4. Verfahren nach einem der vorhergehenden Ansprüche, wobei die genetisch veränderten Lymphozyten CAR-T-Lymphozyten sind.

5. Verfahren nach Anspruch 1, wobei das apoptotische Mittel aus der Gruppe ausgewählt ist, die Taxane einschließlich Docetaxel und Paclitaxel, Anthracycline, Cisplatin, Carboplatin, 5-Fluor-Uracil, Gemcitabin, Capecitabin, Navelbin oder Zoledronat und/oder die Kombination von 8-MOP und UVA umfasst.

6. Verfahren nach Anspruch 1 oder 4, wobei die Monozyten aktiviert und zur Differenzierung in immunstimulierende autologe Antigen-präsentierende Zellen veranlasst werden, indem die Monozyten einer Scherkraft in einer Strömungskammer einer Vorrichtung ausgesetzt werden.

7. Verfahren nach Anspruch 1, 4 oder 5, wobei die Monozyten durch Interaktion mit Thrombozyten und/oder Plasmakomponenten in Abwesenheit von apoptotischen Mitteln aktiviert und zur Differenzierung in immunstimulierende autologe Antigen-präsentierende Zellen veranlasst werden.

8. Verfahren nach Anspruch 1, 4, 5 oder 6, wobei die Krankheitseffektorzellen Zellen sind, die einem Tumor entnommen wurden, und wobei die freigesetzten krankheitsassoziierten Antigene freigesetzte tumorassoziierte Antigene sind.

9. Verfahren nach Anspruch 7, wobei die Tumorzellen aus einem soliden Tumor gewonnen oder von diesem abgesondert werden.

10. Verfahren nach Anspruch 8, wobei der solide Tumor aus der Gruppe ausgewählt ist, die Lungenkrebs, Brustkrebs, Bauchspeicheldrüsenkrebs, Dickdarmkrebs, Prostatakrebs, Kopf- und Halskrebs, Gliom, Hirnkrebs, Eierstock-, Muskel-, Bindegewebs-, Nierenkrebs oder Hautkrebs wie etwa Melanom umfasst.

## Revendications

1. Procédé d'obtention de lymphocytes activés, comprenant au moins les étapes de :
a. fournir des cellules présentant un antigène affichant des antigènes associés à une maladie, les cellules présentant l'antigène étant obtenues par les étapes suivantes de :
i. fournir un échantillon extracorporel de cellules effectrices de maladie mammifères et soumettre lesdites cellules effectrices de maladie à des agents apoptotiques pour libérer des antigènes associés à la maladie ou fournir des antigènes associés à la maladie isolés,
ii. fournir une quantité extracorporelle d'un échantillon de sang d'un sujet mammifère comprenant des monocytes séparément de l'étape i),
iii. activer lesdits monocytes pour induire leur différenciation en APC autologues immunostimulatrices en l'absence d'agents apoptotiques séparément de l'étape i),
iv. combiner lesdites APC autologues immunostimulatrices de l'étape iii) avec lesdites cellules effectrices de maladie apoptotiques ou les antigènes associés à la maladie isolés de l'étape i) ;
alternativement, l'étape iv) pouvant être effectuée avant d'effectuer l'étape iii) de telle manière que lesdites cellules effectrices de maladie apoptotiques ou les antigènes associés à la maladie isolés de l'étape i) étant combinés avec lesdits monocytes de l'étape ii), suivi par l'activation desdits monocytes dans le mélange pour induire leur différenciation en APC autologues immunostimulatrices en l'absence d'agents apoptotiques,
b. fournir une quantité extracorporelle comprenant des lymphocytes T CD8⁺ ou des lymphocytes T génétiquement modifiés ;
c. combiner lesdites cellules présentant un antigène affichant des antigènes associés à la maladie de l'étape a)avec lesdits lymphocytes de l'étape b) ; et
d. incuber le mélange de l'étape c), les cellules présentant un antigène affichant des antigènes associés à la maladie allant activer les lymphocytes pour proliférer.

2. Procédé selon la revendication 1, dans lequel l'incubation de l'étape d) est pour au moins 12 heures, au moins 1 jour, au moins 3 jours, au moins 7 jours ou au moins 14 jours.

3. Procédé selon la revendication 1 ou 2, dans lequel lesdits lymphocytes de l'étape b) sont obtenus d'un échantillon de sujet mammifère, par exemple un échantillon de sang ou de tissu.

4. Procédé selon l'une quelconque des revendications précédentes, dans lequel lesdits lymphocytes génétiquement modifiés sont des lymphocytes T-CAR.

5. Procédé selon la revendication 1, dans lequel l'agent apoptotique est choisi dans le groupe comprenant des taxanes comprenant le docétaxel et le paclitaxel, des anthracyclines, le cisplatine, le carboplatine, le 5-fluoro-uracile, la gemcitabine, la capécitabine, la navelbine ou le zolédronate et/ou la combinaison de 8-MOP et d'UVA.

6. Procédé selon la revendication 1 ou 4, dans lequel lesdits monocytes sont activés et induits à se différencier en cellules présentant un antigène autologues immunostimulatrices en soumettant lesdits monocytes à une force de cisaillement dans une chambre d'écoulement d'un dispositif.

7. Procédé selon les revendications 1, 4 ou 5, dans lequel lesdits monocytes sont activés et induits à se différencier en cellules présentant un antigène autologues immunostimulatrices par l'interaction avec des plaquettes et/ou des composants de plasma en l'absence d'agents apoptotiques.

8. Procédé selon les revendications 1, 4, 5 ou 6, dans lequel lesdites cellules effectrices de maladie sont des cellules prélevées d'une tumeur et lesdits antigènes associés à la maladie libérés étant des antigènes associés à la tumeur.

9. Procédé selon la revendication 7, dans lequel lesdites cellules tumorales sont obtenues de ou partagées d'une tumeur solide.

10. Procédé selon la revendication 8, dans lequel ladite tumeur solide est choisie dans le groupe comprenant le cancer du poumon, le cancer du sein, le cancer pancréatique, le cancer du côlon, le cancer de la prostate, le cancer de la tête et du cou, le gliome, le cancer du cerveau, ovarien, du muscle, du tissu conjonctif, le cancer du rein ou les cancers de la peau tels que le mélanome.
